# EUROPEAN PATENT APPLICATION

(11) **EP 4 424 842 A1**
(43) Date of publication of application: **04.09.2024**
(21) Application number: 23461526.8
(22) Date of filing: 01.03.2023
(51) Int. Cl.: C12Q 1/6841, C12Q 1/6844, C12Q 1/6804

(54) **METHOD FOR DETECTION OF NUCLEIC ACID END(S)**

(71) Applicant: INTODNA SPÓLKA AKCYJNA, 30-348 Kraków (PL)
(72) Inventor: KITLINSKA, Agata, Kraków (PL); SIERPOWSKI, Franek, Kraków (PL); PRUCSI, Zsombor, Kraków (PL); JAROSZ, Monika, Kraków (PL); BIALECKA, Maja, Kraków (PL); UZNANSKA, Karolina, Kraków (PL); WÓJCIKOWSKA, Olga, Kraków (PL); SOLARCZYK, Kamil, Kraków (PL); KORDON-KISZALA, Magdalena, Kraków (PL)
(74) Representative: Boult Wade Tennant LLP

(57) **Abstract**

The invention relates to a method for detection of nucleic acid end(s) in a cell. Specifically, the method relates to a method for detection of nucleic acid end(s) in a circulating tumour cell. The invention also relates to a kit for detection of nucleic acid end(s). Additional methods of the invention relate to assessing effectiveness of a therapeutic agent and assessing a nucleic acid damage caused by an agent.

## Description

### FIELD

The invention relates to a method for detection of nucleic acid end(s) in a cell. Specifically, the method relates to a method for detection of nucleic acid end(s) in a circulating tumour cell. The invention also relates to a kit for detection of nucleic acid end(s). Additional methods of the invention relate to assessing effectiveness of a therapeutic agent and assessing nucleic acid damage caused by an agent.

### BACKROUND

Cancer is one of the terminal diseases that threaten human lives. Cancer metastasis is the primary cause of mortality and accounts for most cancer-related deaths. Circulating tumour cells (CTCs) are tumour cells that shed from the primary tumour and intravasate into the peripheral blood circulation system responsible for metastasis. Targeting and/or neutralization of CTCs in circulation could represent an effective strategy to prevent metastasis. One of the therapeutic strategies in development includes drugs targeting unique cancer markers (e.g. overexpression of surface receptors) found on CTCs. Other strategies aim to alter the tumour-specific microenvironment (e.g. low pH or hypoxia).

When new therapeutic strategies are considered and tested, it is beneficial to detect, visualize, localize, and/or quantify nucleic acid changes in CTCs after administration of a new therapeutic candidate. For example, if the new therapeutic candidate causes damage to nucleic acid(s) in CTCs (e.g. by fragmenting the nucleic acid(s) to generate a plurality of nucleic acid end(s)), this provides a good indication of effectiveness of a therapeutic candidate. CTCs isolated from peripheral blood may be detected using methods known in the art. For example, CTC detection may rely on antibodies which selectively bind to antigens found on the surface of the CTC. The antibodies may be conjugated to magnetic nanoparticles or immobilized on the walls of microfluidic chips for CTC capture through the positive selection of CTC. However, none of the known methods is able to capture the CTCs (or other circulating cells) in a manner such that the nucleic acid end(s) may be detected, visualized, localized and quantified. Thus, a need exists for new tools and methods for validation and/or understanding of the mode of action of new therapeutic candidates targeting circulating cells (e.g. CTCs).

In addition, it is also important to assess the impact of environmental agents (e.g. hazardous environmental agents) on the health status of a subject exposed to said agents. This can be done by detecting, visualizing and/or localizing nucleic acid changes in circulating cells (e.g. blood cells) after exposure to the environment agent. Thus, a need exists for new tools and methods for assessing the level of exposure of the subject to environmental agents.

WO2019/035727 A1 describes a method for detecting DNA end(s). The method relies on molecules, such as BrdU, binding to the DNA end(s), followed by binding of binding molecules (e.g. antibodies) to the molecules bound to the DNA end(s). In the method, the binding molecules are conjugated to oligonucleotides which are able to hybridize with further oligonucleotides to form a circular structure. The circular structure provides a template for rolling circle amplification. Once amplified, the amplification product may be detected by detection methods known in the art, for example, fluorescence microscopy. HeLa cells, which are used in WO2019/035727 A1 as a model cell line for testing detection DNA end(s), belong to a family of immobilized cell lines. Thus, there is no need to attach and/or immobilize those cells prior to detection. A need exists, therefore, for a method of immobilizing other cell lines (e.g. suspension cells or adhesive cells) on a solid support prior to detection of nucleic end(s).

### SUMMARY OF THE INVENTION

The invention provides a method for detection of nucleic acid end(s) in a cell. The method may comprise the steps of:
a) attaching a cell from a suspension of cells to a hydrophilic solid support; and
b) performing detection of nucleic acid end(s) according to a method as described in WO2019/035727.

The method may comprise the steps of:
a) attaching a cell from a suspension of cells to a hydrophilic solid support;
b) adding binding molecules that bind to nucleic acid binding molecules, wherein the nucleic acid binding molecules are (i) bound to the nucleic acid end(s); and/or (ii) present in the proximity of the nucleic acid ends; wherein the molecules are attached to oligonucleotide molecules;
c) adding further oligonucleotide molecules which hybridise with the oligonucleotide molecules attached to the molecules to form a circular amplification template;
d) performing amplification to produce an amplification product;
e) detecting the amplification product to detect the nucleic acid end(s) in the cell.

The method may comprise the steps of:
a) attaching a cell from a suspension of cells to a hydrophilic solid support;
b) adding nucleic acid binding molecules to the cell attached to the solid support so that the nucleic acid binding molecules bind to the nucleic acid end(s) in the cell;
c) adding binding molecules that bind to the nucleic acid binding molecules bound to the nucleic acid end(s), wherein the molecules are attached to oligonucleotide molecules;
d) adding further oligonucleotide molecules which hybridise with the oligonucleotide molecules attached to the molecules to form a circular amplification template;
e) performing nucleic acid amplification to produce an amplification product;
f) detecting the amplification product to detect the nucleic acid end(s) in the cell.

The method may comprise the steps of:
a) attaching a cell from a suspension of cells to a hydrophilic solid support;
b) adding nucleic acid binding molecules to the cell attached to the solid support under conditions such that the nucleic acid binding molecules bind to the nucleic acid end(s) in the cell;
c) adding two different binding molecules that bind to the nucleic acid binding molecules bound to the nucleic acid end(s), wherein each of the two binding different molecules is attached to an oligonucleotide molecule;
d) adding further oligonucleotide molecules which hybridise with the oligonucleotide molecules attached to the two different binding molecules to form a circular amplification template;
e) performing nucleic acid amplification to produce an amplification product;
f) detecting the amplification product to detect the nucleic acid end(s) in the cell.

The invention provides a method for detection of nucleic acid end(s) in a cell, the method comprising the steps of:
a) attaching a cell from a suspension of cells to a hydrophilic solid support under conditions such that the suspension of cells does not dry out;
b) adding nucleic acid binding molecules to the cell attached to the solid support under conditions such that the nucleic acid binding molecules bind to the nucleic acid end(s) in the cell;
c) adding two different binding molecules that bind to the nucleic acid binding molecules bound to the nucleic acid end(s), wherein each of the two different binding molecules is attached to an oligonucleotide molecule;
d) adding further oligonucleotide molecules which hybridise with the oligonucleotide molecules attached to the two different binding molecules to form a circular amplification template;
e) performing nucleic acid amplification to produce an amplification product;
f) detecting the amplification product to detect the nucleic acid end(s) in the cell.

Preferably, the hydrophilic solid support is a positively charged solid support.

The invention provides a method for detection of nucleic acid end(s) in a circulating tumour cell, wherein the method comprises the steps of:
a) attaching a circulating tumour cell from a suspension of circulating tumour cells to a hydrophilic solid support under conditions such that the suspension of circulating tumour cells does not dry out;
b) adding nucleic acid binding molecules to the cell attached to the solid support so that the nucleic acid binding molecules bind to the nucleic acid end(s) in the cell;
c) adding two different binding molecules that bind to the nucleic acid binding molecules bound to the nucleic acid end(s), wherein each of the two different binding molecules is attached to an oligonucleotide molecule;
d) adding further oligonucleotide molecules which hybridise with the oligonucleotide molecules attached to the two different binding molecules to form a circular amplification template;
e) performing nucleic acid amplification to produce an amplification product; and
f) detecting the amplification product to detect the nucleic acid end(s) in the cell.

The invention provides a method for detection of nucleic acid end(s) in a cell, wherein the method comprises the steps of:
a) attaching a cell from a suspension of cells to a hydrophilic solid support under conditions such that the suspension of cells does not dry out;
b) removing the suspension of cells to remove unattached cells;
c) fixing the cell attached to the solid support;
d) adding nucleic acid binding molecules to the cell attached to the solid support so that the nucleic acid binding molecules bind to the nucleic acid end(s) in the cell;
e) adding two different binding molecules that bind to the nucleic acid binding molecules bound to the nucleic acid end(s), wherein each of the two different binding molecules is attached to an oligonucleotide molecule;
f) adding further oligonucleotide molecules which hybridise with the oligonucleotide molecules attached to the two different binding molecules to form a circular amplification template;
g) performing nucleic acid amplification to produce an amplification product; and
h) detecting the amplification product to detect the nucleic acid end(s) in the cell.

The invention provides a method for detection of nucleic acid end(s) in a cell, wherein the method comprises the steps of:
a) attaching a cell from a suspension of cells to a hydrophilic solid support under conditions such that the suspension of cells does not dry out;
b) removing the suspension of cells to remove unattached cells;
c) fixing the cell attached to the solid support;
d) storing the cell at a temperature of between -30 °C to -15°C for at least 4 hours;
e) adding nucleic acid binding molecules to the cell attached to the solid support so that the nucleic acid binding molecules bind to the nucleic acid end(s) in the cell;
f) adding two different binding molecules that bind to the nucleic acid binding molecules bound to the nucleic acid end(s), wherein each of the two different binding molecules is attached to an oligonucleotide molecule;
g) adding further oligonucleotide molecules which hybridise with the oligonucleotide molecules attached to the two different binding molecules to form a circular amplification template;
h) performing nucleic acid amplification to produce an amplification product; and
i) detecting the amplification product to detect the nucleic acid end(s) in the cell.

In another aspect, the invention provides a kit for detection of nucleic acid end(s) in a cell, the kit comprising:
a) a hydrophilic solid support;
b) nucleic acid binding molecules;
c) binding molecules that bind to the nucleic acid binding molecules; and
d) further oligonucleotide molecules.

The kit for detection of nucleic acid end(s) in a cell may comprise:
a) a hydrophilic solid support;
b) nucleic acid binding molecules;
c) two different binding molecules that bind to the nucleic acid binding molecules;
d) further oligonucleotide molecules; and
e) reagents for performing nucleic acid amplification.

In an aspect, the invention provides a method for assessing effectiveness of a therapeutic agent, the method comprising the steps:
a) performing the method for detection of nucleic acid end(s) as described herein on a suspension of circulating tumour cells obtained from a subject before the administration of the therapeutic agent,
b) performing the method for detection of nucleic acid end(s) as described herein on a suspension of circulating tumour cells obtained from the subject after the administration of the therapeutic agent,
c) comparing the amount, and optionally the cellular location, of the nucleic acid end(s) detected in steps a) and b), wherein the therapeutic agent is effective if the amount of the nucleic acid end(s) is greater in step b) than step a),
wherein steps a) and b) can be performed in any order.

In an aspect, the invention provides a method for assessing nucleic acid damage caused by an agent, the method comprising the steps:
a) performing the method for detection of nucleic acid end(s) as described herein on a suspension of cells obtained from a subject before administration of or exposure to an agent,
b) performing the method for detection of nucleic acid end(s) as described herein on a suspension of cells obtained from the subject after the administration of or exposure to the agent,
c) comparing the amount, and optionally the cellular location, of the nucleic acid end(s) detected in steps a) and b), wherein the agent causes nucleic acid damage if the amount of the nucleic acid end(s) is greater in step b) than step a),
wherein steps a) and b) can be performed in any order.

Each aspect or embodiment as defined herein may be combined with any other aspect(s) or embodiment(s) unless clearly indicated to the contrary. In particular any feature indicated as being preferred or advantageous may be combined with any other feature or features indicated as being preferred or advantageous.

The present inventors have surprisingly discovered that the use of a hydrophilic (e.g. positively charged) solid support allows for an efficient immobilization and/or attachment of adherent and suspension cells. In addition, the inventors have determined attachment conditions which enable efficient detection of nucleic acid end(s) in the cell. For example, attachment under wet conditions (i.e. conditions which prevent drying out of the cells) retains the highest number of cells attached to a solid support, allowing for a more accurate nucleic acid end(s) detection.

### DETAILED DESCRIPTION

Unless otherwise defined herein, scientific, and technical terms used in connection with the present invention shall have the meanings that are commonly understood by those of ordinary skill in the art. The meaning and scope of the terms should be clear, however, in the event of any latent ambiguity, definitions provided herein take precedent over any dictionary or extrinsic definition.

It should be understood that singular prepositions such as "a," "an," and "the," are often used for convenience, however, all instances of the singular are intended to encompass the plural unless otherwise indicated either explicitly or from context. The terms "comprising", "including" and "having" are intended to be inclusive and mean that there may be additional elements other than the listed elements. Further, it should be understood that all references, including journal articles, books, patents, technical documents, and the like, mentioned in this disclosure are hereby incorporated by reference in their entirety and for all purposes.

The term "about" as used herein for numerical data refers to a value within 10% of the underlying parameter (i.e., plus or minus 10%), and use of the term "about" at the beginning of a string of values modifies each of the values (i.e., "about 1, 2 and 3" refers to about 1, about 2 and about 3). For example, a temperature of "about 25 °C" can include temperatures between (and including) 22.5 °C and 27.5 °C.

The term "wet attachment" as used herein refers to a method of attaching cells under conditions such that the cells are not dried out. This may be accomplished by different methods known in the art, for example, by placing the cells in the humidity chamber or CO2 incubator, or by adjusting the temperature so that the cells remain in solution throughout the duration of the attachment.

The term "dry attachment" as used herein refers to methods of attaching cells which rely on drying the cells. For example, dry attachment may include a smear on a solid support.

The invention provides a method for detection of nucleic acid end(s) in a cell.

The method may comprise the steps of:
a) attaching a cell from a suspension of cells to a hydrophilic solid support; and
b) performing detection of nucleic acid end(s) according to a method as described in WO2019/035727.

WO2019/035727 A1 is hereby incorporated by reference in its entirety and for all aspects and embodiments described herein.

The method may comprise the steps of:
a) attaching a cell from a suspension of cells to a hydrophilic solid support;
b) adding binding molecules that bind to nucleic acid binding molecules, wherein the nucleic acid binding molecules are (i) bound to the nucleic acid end(s); and/or (ii) present in the proximity of the nucleic acid ends; wherein the binding molecules are attached to oligonucleotide molecules;
c) adding further oligonucleotide molecules which hybridise with the oligonucleotide molecules attached to the binding molecules to form a circular amplification template;
d) performing nucleic acid amplification to produce an amplification product;
e) detecting the amplification product to detect the nucleic acid end(s) in the cell.

The method may comprise the steps of:
a) attaching a cell from a suspension of cells to a hydrophilic solid support;
b) adding nucleic acid binding molecules to the cell attached to the solid support under conditions such that the nucleic acid binding molecules bind to the nucleic acid end(s) in the cell;
c) adding binding molecules that bind to the nucleic acid binding molecules bound to the nucleic acid end(s), wherein the binding molecules are attached to oligonucleotide molecules;
d) adding further oligonucleotide molecules which hybridise with the oligonucleotide molecules attached to the binding molecules to form a circular amplification template;
e) performing nucleic acid amplification to produce an amplification product; and
f) detecting the amplification product to detect the nucleic acid end(s) in the cell.

The method may rely on two different binding molecules that bind to the nucleic acid end(s) in the cell. Thus, the method may comprise the steps of:
a) attaching a cell from a suspension of cells to a hydrophilic solid support;
b) adding nucleic acid binding molecules to the cell attached to the solid support under conditions such that the nucleic acid binding molecules bind to the nucleic acid end(s) in the cell;
c) adding two different binding molecules that bind to the nucleic acid binding molecules bound to the nucleic acid end(s), wherein each of the two different binding molecules is attached to an oligonucleotide molecule;
d) adding further oligonucleotide molecules which hybridise with the oligonucleotide molecules attached to the two different binding molecules to form a circular amplification template;
e) performing nucleic acid amplification to produce an amplification product;
f) detecting the amplification product to detect the nucleic acid end(s) in the cell.

The method may further rely on a wet attachment of the cells to the solid support. Thus, the invention provides a method for detection of nucleic acid end(s) in a cell, the method comprising the steps of:
a) attaching a cell from a suspension of cells to a hydrophilic solid support under conditions such that the suspension of cells does not dry out;
b) adding nucleic acid binding molecules to the cell attached to the solid support under conditions such that the nucleic acid binding molecules bind to the nucleic acid end(s) in the cell;
c) adding two different binding molecules that bind to the nucleic acid binding molecules bound to the nucleic acid end(s), wherein each of the two different binding molecules is attached to an oligonucleotide molecule;
d) adding further oligonucleotide molecules which hybridise with the oligonucleotide molecules attached to the two different binding molecules to form a circular amplification template;
e) performing amplification to produce an amplification product;
f) detecting the amplification product to detect the nucleic acid end(s) in the cell.

Wet attachment is preferred as it enables attachment of the optimal number of cells for the nucleic acid end(s) detection method.

The hydrophilic solid support may be positively charged or negatively charged. Preferably, the hydrophilic solid support is a positively charged solid support. Thus, the method may comprise the steps:
a) attaching a cell from a suspension of cells to a positively charged solid support under conditions such that the suspension of cells does not dry out;
b) adding nucleic acid binding molecules to the cell attached to the solid support under conditions such that the nucleic acid binding molecules bind to the nucleic acid end(s) in the cell;
c) adding two different binding molecules that bind to the nucleic acid binding molecules bound to the nucleic acid end(s), wherein each of the two different binding molecules is attached to an oligonucleotide molecule;
d) adding further oligonucleotide molecules which hybridise with the oligonucleotide molecules attached to the two different binding molecules to form a circular amplification template;
e) performing amplification to produce an amplification product;
f) detecting the amplification product to detect the nucleic acid end(s) in the cell.

The inventors have found that the use of a hydrophilic (e.g. positively charged) solid support significantly improves the attachment of cells in suspension. With reference to Example 1, the inventors have tested a large number of solid supports (including a glass slide, a coverslip, a 96-well plate, and slides with different coatings) and found that only solid supports (e.g. slides) with a hydrophilic coating (e.g. positively charged slides) enable efficient attachment of cells from suspension of cells.

By hydrophilic solid support is meant a solid support that comprises at least one hydrophilic outer surface. Thus, a hydrophilic surface is presented to the suspension of cells resulting in attachment of individual cell(s) from the suspension to the solid support (via the hydrophilic surface). The hydrophilic solid support may be a slide or a coverslip. The hydrophilic nature of the solid support may result from a coating on the solid support or from the composition of the solid support itself. The coating may provide a positive or negative charged surface, but is preferably a positive charge. For example, the solid support may be a TOMO^{®} slide or a poly-L-lysine coated slide, both of which provide a positively charged surface to which cells bind. The solid support may be a silicate uncoated glass slide in some embodiments.

The method described herein may detect any type of nucleic acid end(s). For example, the nucleic acid end(s) may be DNA end(s), RNA end(s), or DNA:RNA hybrid end(s). Preferably, the nucleic acid end(s) are DNA end(s).

The method described herein is sensitive and/or specific enough to detect single stranded and double stranded nucleic acid end(s). Thus, the nucleic acid end(s) may be single-stranded end(s) and/or double-stranded end(s).

The high sensitivity and/or specificity of the method also allows for the detection of a single nucleic acid end in a cell. Thus, the nucleic acid end(s) may be a single nucleic acid end.

The method described herein is useful in the attachment of an adherent cell and/or a suspension cell. Thus, the cell may be an adherent cell and/or a suspension cell. The method may be used in the attachment of any cell which is in solution prior to the detection of the nucleic acid end(s). Preferably, the method is used in the attachment of a suspension cell. The attachment cell is anchorage-dependent and has to be cultured on a suitable substrate that is specifically treated to allow cell adhesion and spreading. Suspension cell lines are the cell lines in which the cultures are suspended in liquid media, and the cells thus remain in the fluid media. They are not anchorage dependent, which makes them particularly difficult to attach to a solid support. The inventios of the present application overcame this difficulty by applying the attachment step as described herein.

The majority of the cells derived from vertebrates, with the exception of hematopoietic cell lines and a few others, are anchorage-dependent. Thus, the method for detection of nucleic acid end(s) may detect nucleic acid end(s) in a blood cell. In a subject suffering from cancer, certain tumour cells may also belong to a family of suspension cells. For example, the cell may be a circulating tumour cell. Thus, the method for detection of nucleic acid end(s) may detect nucleic acid end(s) in circulating tumour cells. The circulating tumour cells may include a mixed population of cells or they me be pre-purified.

Detection of nucleic acid end(s) in cell(s) derived from a subject suffering from cancer may be performed. Preferably, the cell(s) are cancer cell(s), such as circulating tumour cell(s). The cancer may be of any cancer type found in the body. The cancer may be a breast cancer, a prostate cancer, a kidney cancer, a bladder cancer, a colorectal cancer, a non-small-cell lung cancer, a pancreatic cancer, a gastric cancer, a cervical cancer or melanoma. The method may be used in the detection of cells originating from cancer types which form metastasis. Metastasis is facilitated by circulating tumour cell(s). Thus, the method preferably detects nucleic acid end(s) in circulating tumour cells forming metastasis.

The method may detect nucleic acid end(s) in 22Rv1 or LNCaP cell lines.

The step of attaching the cell may be performed at a temperature of between 10 °C to 40 °C, or between 15 °C to 37 °C. For example, the step of attaching the cell may be performed at a temperate of between 20 °C to 27 °C, or between 34 °C to 40 °C. The step of attaching the cell may be performed at a temperature of about 25 °C or about 37 °C.

The step of attaching the cell may be performed for between 30 min to 4 hours, or between 1 hour to 3 hours. The step of attaching the cell is preferably performed for about 2 hours. The step of attaching the cell may be performed for at least 30 min, at least 1 hour, at least 90 minutes, at least 2 hours, at least 2.5 hours or at least 3 hours.

The step of attaching the cell may be performed in a humidity chamber and/or a CO2 incubator. The step of attaching the cell may be performed under any conditions in which the cell does not dry out during the attaching step. That is to say that the step of attaching the cell ensures that the cell remains substantially surrounded by liquid throughout the step (allowing for the fact that at least part of the cell becomes attached to the hydrophilic solid support during this step). The attaching step may be performed under conditions such that, during the attaching step, the suspension of cells retains at least 95%, at least 90%, at least 85%, at least 80%, at least 75%, at least 70%, at least 65%, at least 60%, at least 55%, at least 50%, at least 45%, at least 40%, at least 35%, at least 30%, at least 25%, or at least 20% of the initial volume of the suspension of cells applied to the solid support before (or at the start of) the attaching step. The step of attaching the cell may be performed under conditions such that at least 10%, at least 20%, at least 30%, at least 40%, at least 50%, at least 60%, at least 70%, at least 80%, at least 90%, or 100% of the liquid from the suspension of cells is retained during the step of attaching the cell. For example, the step of attaching the cell may be performed under conditions such that at least 5 µL, at least 10 µL, at least 20 µL, at least 30 µL, at least 40 µL, at least 50 µL, at least 60 µL, at least 70 µL, at least 80 µL, at least 90 µL, or at least 100 µL of the liquid content of the suspension of cells is retained at the end of the attaching step. The liquid or liquid content may comprise water, a buffer and/or a medium. The step of attaching the cell may be performed under conditions such that less than 1%, less than 3%, less than 5%, less than 10%, less than 20%, less than 30%, less than 40%, less than 50%, less than 60%, less than 70%, less than 80%, or less than 90% of the liquid from the suspension of cells is lost during the attaching step. The step of attaching the cell may be performed under conditions such that the cell remains viable during the attaching step. The step of attaching the cell may be performed with the help of any tools or devices that prevent the cell from drying out during the step of attaching.

In the step of attaching, the suspension of cells may have a density of between 0.1 × 10⁶ to 4 × 10⁶ cells/ml. Preferably, the suspension of cells has a density of between 1.5 × 10⁶ to 2 × 10⁶ cells/ml. Prior to the step of attachment, the cells in the suspension of cells may be counted, for example, in a Burker chamber. For example, the cell density may be adjusted to between 50,000 to 300,000 cells per 100µL prior to the step of attaching. The cell density may be adjusted to between 1.5 × 10⁶ to 2 × 10⁶ cells/ml prior to the step of attaching. The cell density may be adjusted by spinning the cells and re-suspending the cell pellet in a desired volume (e.g. 100µL) of a buffer (e.g. PBS).

The suspension of cells may be applied to a predetermined area of the solid support. For example, a square or circular zone may be outlined prior to the application of the suspension of cells. For example, if the solid support is a slide, a 2cm x 2cm zone may be outlined with a hydrophobic pen. This allows to better visualise the detection zone on the slide.

Prior to the step of attaching, the suspension of cells may be cultured to achieve a desired density. For example, the density may be 1 × 10⁶ to 4 × 10⁶ cells/ml. Preferably, the suspension of cells is cultured to a density of between 1.5 × 10⁶ to 2 × 10⁶ cells/ml. Depending on the cell type, after cultivation, the cells may be detached from the support on which they were cultured (e.g. a flask). The step of detachment may be performed with a protease (e.g. trypsin). The protease may be neutralized prior to the step of attachment.

The suspension of cells may comprise a medium and/or a buffer. The buffer may be PBS.

The cell may be fixed before or after the step of attaching the cell from the suspension of cells. Thus, the method may comprise the steps of:
a) attaching a cell from a suspension of cells to a hydrophilic solid support under conditions such that the suspension of cells does not dry out;
b) fixing the cell attached to the solid support;
c) adding nucleic acid binding molecules to the cell attached to the solid support under conditions such that the nucleic acid binding molecules bind to the nucleic acid end(s) in the cell;
d) adding two different binding molecules that bind to the nucleic acid binding molecules bound to the nucleic acid end(s), wherein each of the two different binding molecules is attached to an oligonucleotide molecule;
e) adding further oligonucleotide molecules which hybridise with the oligonucleotide molecules attached to the two different binding molecules to form a circular amplification template;
f) performing amplification to produce an amplification product; and
g) detecting the amplification product to detect the nucleic acid end(s) in the cell.

The method may comprise the steps of:
a) fixing a cell from a suspension of cells;
b) attaching the cell from a suspension of cells to a hydrophilic solid support under conditions such that the suspension of cells does not dry out;
c) adding nucleic acid binding molecules to the cell attached to the solid support under conditions such that the nucleic acid binding molecules bind to the nucleic acid end(s) in the cell;
d) adding two different binding molecules that bind to the nucleic acid binding molecules bound to the nucleic acid end(s), wherein each of the two different binding molecules is attached to an oligonucleotide molecule;
e) adding further oligonucleotide molecules which hybridise with the oligonucleotide molecules attached to the two different binding molecules to form a circular amplification template;
f) performing amplification to produce an amplification product; and
g) detecting the amplification product to detect the nucleic acid end(s) in the cell.

The step of fixation is beneficial because it allows to preserve the cells and store them for a longer period of time. The step of fixation also allows to preserve nucleic acid end(s). The step of fixation may also preserve an antigen of interest for distinction between cell types (e.g. circulating tumour cell types).

The step of fixation may be performed in 70%(v/v) ethanol. The step of fixation may be performed for between 1 h to 24 hrs, 2 hrs to 12 hrs, or 3 hrs to 6 hrs. Preferably, the step of fixation is performed for at least 2 hrs. The step of fixation may be performed for at least 8 hrs. The step of fixation may be performed at a temperature of between -80 °C to 30°C, between -40 °C to 25°C, or between -20 °C to 25°C. For example, the step of fixation may be performed at about 22 °C (i.e. room temperature) or at 0 °C. For example, the step of fixation may be performed at about 22 °C (i.e. room temperature) for at least 2 hours prior to the attachment of cells to the solid support.

In embodiments in which the step of fixation is performed after the step of attaching, after the cells have been attached to the solid support, remaining solution (which may include suspension of cells, buffer and/or medium) is removed. Once the remaining solution is removed, the attached cell(s) may be fixed in 70%(v/v) ethanol. Ethanol is preferably ice-cold (i.e. has a temperature of between 0 °C to 4 °C). After application of ethanol, the cells may be stored at between -80 °C to 20°C, between -40 °C to 10°C, or between 20 °C to 4°C. Preferably, the cells are stored at about -20 °C. After application of ethanol, the cells may be stored for at least 1 hour, at least 2 hours, at least 3 hours or at least 4 hours. Preferably, the cells are stored for at least 4 hours at about -20 °C.

In embodiments in which the step of fixation is performed before the step of attaching, the suspension of cells may be cultured to achieve a desired density. For example, the density may be.1 × 10⁶ to 4 × 10⁶ cells/ml. Preferably, the suspension of cells is cultured to a density of between 1.5 × 10⁶ to 2 × 10⁶ cells/ml. Depending on the cell type, after cultivation, the cells may be detached from the support on which they were cultured (e.g. a flask). The step of detachment may be performed with a protease (e.g. trypsin). The protease may be neutralized prior to the step of fixation. Prior to the step of fixation, the cells may be counted, for example, in a Burker chamber. For example, the cell density may be adjusted to between 100,000 to 300,000 cells in 10 mL prior to the step of fixing. The cells may be in suspension, for example in a medium or in a buffer. Prior to the step of fixing, the cells may be centrifuged to obtain a cell pellet. The step of fixation may be performed on the cell pellet. Thus, the cell pellet may be resuspended in ethanol during the step of fixation. Preferably, the cell pellet is resuspended in 70%(v/v) ethanol. Ethanol is preferably ice-cold (i.e. has a temperature of between 0 °C to 4 °C). After application of ethanol, the cells may be stored at between -80 °C to 20°C, between -40 °C to 10°C, or between 20 °C to 4°C. Preferably, the cells are stored at about -20 °C. After application of ethanol, the cells may be stored for at least 1 hour, at least 2 hours, at least 3 hours or at least 4 hours. Preferably, the cells are stored for at least 4 hours at about -20 °C. After application of ethanol, the cells may be incubated with ethanol for at least 1 hour, at least 1.5 hour, at least 2 hours, at least 3 hours, or at least 4 hours. When fixation is performed before attachment, the cells may be first fixed in 70% (v/v) ethanol for at least 1 hour, at least 2 hours, at least 4 hours, at least 6 hours, or at least 8 hours at about 22 °C (i.e. room temperature), and then attached to the solid support. In this embodiment, the use of a humidity chamber or CO2 incubator is not required. In this embodiment, the step of attachment may be carried out at about 22 °C (i.e. room temperature).

In the methods described herein, the suspension of cells, medium, buffer, and/or any other solution may be removed from the solid support after the step of attaching the cell and before the step of adding nucleic acid binding molecules. This allows to remove any unattached cells from the solid support to reduce the background signal/noise when performing the detection method. The step of removing the suspension of cells may include separating the solid support from the suspension of cells. Thus, the method for detection of nucleic acid end(s) in a cell may comprise the steps of:
a) attaching a cell from a suspension of cells to a hydrophilic solid support under conditions such that the suspension of cells does not dry out;
b) removing the suspension of cells to remove unattached cells;
c) adding nucleic acid binding molecules to the cell attached to the solid support under conditions such that the nucleic acid binding molecules bind to the nucleic acid end(s) in the cell;
d) adding two different binding molecules that bind to the nucleic acid binding molecules bound to the nucleic acid end(s), wherein each of the two different binding molecules is attached to an oligonucleotide molecule;
e) adding further oligonucleotide molecules which hybridise with the oligonucleotide molecules attached to the two different binding molecules to form a circular amplification template;
f) performing amplification to produce an amplification product; and
g) detecting the amplification product to detect the nucleic acid end(s) in the cell.

The method for detection of nucleic acid end(s) in a cell may comprise the steps of:
a) attaching a cell from a suspension of cells to a hydrophilic solid support under conditions such that the suspension of cells does not dry out;
b) removing the suspension of cells to remove unattached cells;
c) fixing the cell attached to the solid support;
d) adding nucleic acid binding molecules to the cell attached to the solid support under conditions such that the nucleic acid binding molecules bind to the nucleic acid end(s) in the cell;
e) adding two different binding molecules that bind to the nucleic acid binding molecules bound to the nucleic acid end(s), wherein each of the two different binding molecules is attached to an oligonucleotide molecule;
f) adding further oligonucleotide molecules which hybridise with the oligonucleotide molecules attached to the two different binding molecules to form a circular amplification template;
g) performing amplification to produce an amplification product; and
h) detecting the amplification product to detect the nucleic acid end(s) in the cell.

After certain method steps described herein the cell(s) may be stored. The cell(s) may be stored at a temperature of between -80 °C to -4°C, or between -30 °C to -15°C. For example, the cell(s) may be stored after the step of attaching and before the step of adding nucleic acid binding molecules. For example, the cell(s) may be stored after the step of fixation and before the step of adding nucleic acid binding molecules. For example, the cell(s) may be stored after the step of fixation and before the step of attaching. Preferably, the cell(s) are stored at a temperature of about - 20°C. The cell(s) may be stored for at least 2 hours, at least 3 hours, at least 4 hours, at least 5 hours, or at least 6 hours, preferably at least 4 hours. Thus, the method for detection of nucleic acid end(s) in a cell may comprise the steps of:
a) attaching a cell from a suspension of cells to a hydrophilic solid support under conditions such that the suspension of cells does not dry out;
b) fixing the cell attached to the solid support;
c) storing the cell at a temperature of between -30 °C to -15°C for at least 4 hrs;
d) adding nucleic acid binding molecules to the cell attached to the solid support under conditions such that the nucleic acid binding molecules bind to the nucleic acid end(s) in the cell;
e) adding two different binding molecules that bind to the nucleic acid binding molecules bound to the nucleic acid end(s), wherein each of the two different binding molecules is attached to an oligonucleotide molecule;
f) adding further oligonucleotide molecules which hybridise with the oligonucleotide molecules attached to the two different binding molecules to form a circular amplification template;
g) performing amplification to produce an amplification product;
h) detecting the amplification product to detect the nucleic acid end(s) in the cell.

The method for detection of nucleic acid end(s) in a cell may comprise the steps of:
a) attaching a cell from a suspension of cells to a hydrophilic solid support under conditions such that the suspension of cells does not dry out;
b) removing the suspension of cells to remove unattached cells;
c) fixing the cell attached to the solid support;
d) storing the cell at a temperature of between -30 °C to -15°C for at least 4 hrs;
e) adding nucleic acid binding molecules to the cell attached to the solid support under conditions such that the nucleic acid binding molecules bind to the nucleic acid end(s) in the cell;
f) adding two different binding molecules that bind to the nucleic acid binding molecules bound to the nucleic acid end(s), wherein each of the two different binding molecules is attached to an oligonucleotide molecule;
g) adding further oligonucleotide molecules which hybridise with the oligonucleotide molecules attached to the two different binding molecules to form a circular amplification template;
h) performing amplification to produce an amplification product; and
i) detecting the amplification product to detect the nucleic acid end(s) in the cell.

The method is preferably used to detect nucleic acid end(s) in a circulating tumour cell. Thus, the invention provides a method for detection of nucleic acid end(s) in a circulating tumour cell, wherein the method comprises the steps of:
a) attaching a circulating tumour cell from a suspension of circulating tumour cells to a hydrophilic solid support under conditions such that the suspension of circulating tumour cells does not dry out;
b) adding nucleic acid binding molecules to the cell attached to the solid support under conditions such that the nucleic acid binding molecules bind to the nucleic acid end(s) in the cell;
c) adding two different binding molecules that bind to the nucleic acid binding molecules bound to the nucleic acid end(s), wherein each of the two different binding molecules is attached to an oligonucleotide molecule;
d) adding further oligonucleotide molecules which hybridise with the oligonucleotide molecules attached to the two different binding molecules to form a circular amplification template;
e) performing amplification to produce an amplification product; and
f) detecting the amplification product to detect the nucleic acid end(s) in the cell.

Preferably, the hydrophilic solid support is a positively charged solid support.

The method may comprise the steps:
a) attaching a circulating tumour cell from a suspension of circulating tumour cells to a hydrophilic solid support under conditions such that the suspension of circulating tumour cells does not dry out;
b) fixing the circulating tumour cell attached to the solid support;
c) adding nucleic acid binding molecules to the cell attached to the solid support under conditions such that the nucleic acid binding molecules bind to the nucleic acid end(s) in the cell;
d) adding two different binding molecules that bind to the nucleic acid binding molecules bound to the nucleic acid end(s), wherein each of the two different binding molecules is attached to an oligonucleotide molecule;
e) adding further oligonucleotide molecules which hybridise with the oligonucleotide molecules attached to the two different binding molecules to form a circular amplification template;
f) performing amplification to produce an amplification product; and
g) detecting the amplification product to detect the nucleic acid end(s) in the cell.

The method may comprise the steps:
a) fixing the circulating tumour cell from a suspension of circulating tumour cells;
b) attaching the fixed circulating tumour cell to a hydrophilic solid support under conditions such that the suspension of circulating tumour cells does not dry out;
c) adding nucleic acid binding molecules to the cell attached to the solid support under conditions such that the nucleic acid binding molecules bind to the nucleic acid end(s) in the cell;
d) adding two different binding molecules that bind to the nucleic acid binding molecules bound to the nucleic acid end(s), wherein each of the two different binding molecules is attached to an oligonucleotide molecule;
e) adding further oligonucleotide molecules which hybridise with the oligonucleotide molecules attached to the two different binding molecules to form a circular amplification template;
f) performing amplification to produce an amplification product; and
g) detecting the amplification product to detect the nucleic acid end(s) in the cell.

The nucleic acid binding molecules used in the methods and kits described herein may be any molecules capable of binding to the end of a nucleic acid molecule. The nucleic acid binding molecules used in the methods and kits described herein may also be able to bind to binding molecules (e.g. antibodies or fragments thereof) which are attached (or attachable) to an oligonucleotide molecule. Preferably, the nucleic acid binding molecules use a first binding site to interact and/or bind to the nucleic acid end(s) and a second binding site to interact and bind/or to the binding molecules which are attached to an oligonucleotide molecule. The nucleic acid binding molecules may have different affinity for two different binding molecules which are attached to the oligonucleotide molecule. The nucleic acid binding molecules may be selected from a group comprising:
(i) halogenated nucleotide or nucleoside molecules, such as BrdU, IdU, CldU,
(ii) DNA precursor analoguess such as EdU (5-Ethynyl-2'-deoxyuridine), F-ara-EdU, 5-Ethynyl-2'-deoxycytidine,
(iii) biotinylated nucleotide molecules,
(iv) ADP-ribose molecules,
(v) protein molecules,
(vi) nucleotide, or nucleoside molecules labelled with labels; optionally wherein the labels are selected from a group comprising fluorescent molecules, or chemiluminescent molecules, or radioisotopes, or enzyme substrates, or biotin molecules.

Preferably, the nucleic acid binding molecules are halogenated nucleotide or nucleoside molecules, DNA precursor analogues, and/or biotinylated nucleotide molecules.

The nucleic acid binding molecules may be of the same type or of at least two, at least three, at least four or at least five different types. For example, the nucleic acid binding molecules may be halogenated nucleotides of the same type (e.g. BrdU), or halogenated nucleotides of a different type (e.g. BrdU and IdU). The nucleic acid binding molecules may be halogenated nucleotide and biotinylated nucleotide molecules. The nucleic acid binding molecules may be biotinylated nucleotide molecules of different types (e.g. biotin- ATP, biotin-dGTP, biotin- dUTP, and/or biotin dCTP).

The nucleic acid binding molecules may bind to the nucleic acid end(s) by catalytic or noncatalytic means. The catalytic means may comprise non-enzymatic or enzymatic means. The enzymatic means may comprise DNA polimerase I, TdT, Klenow fragment, Phu polymerase, Taq polymerase, T4 DNA Polymerase, T7 DNA Polymerase, T4 Polynucleotide Kinase, RNA polymerases. The non-enzymatic means may comprise chemical factors, for example chemical factors with catalytic properties. The noncatalytic means may comprise physical or biochemical factors.

The method described herein relies on adding binding molecules that bind to the nucleic acid binding molecules bound to the nucleic acid end(s). Preferably, the binding molecules are two different binding molecules. The binding molecules may be (or comprise) antibodies or fragments thereof, streptavidin molecules, avidin molecules, streptavidin analogues, biotin molecules, peptides, proteins, nucleic acids, azides, or polymers. In embodiments in which the binding molecules are two different binding molecules, the binding molecules may be of the same time or of a different type. For example, the two different binding molecules may be (or comprise):
(i) different antibodies or fragments thereof, such as a monoclonal antibody (or a fragment thereof) and a polyclonal antibody (or a fragment thereof);
(ii) different antibodies or fragments thereof, such as a monoclonal antibody (or a fragment thereof) specific to a first nucleic acid binding molecule used in the method, and a monoclonal antibody (or a fragment thereof) specific to a second nucleic acid binding molecule used in the method;
(iii) an antibody or a fragment thereof and a streptavidin,
(iv) an antibody or a fragment thereof and an avidin,
(v) an antibody or a fragment thereof and a protein or peptide; or
(vi) an antibody or a fragment thereof and a biotin molecule.

The binding molecules that bind to the nucleic acid binding molecules may also be attached to an oligonucleotide molecule. If two different binding molecules are used, each one of the binding molecules is attached to an oligonucleotide molecule. Preferably, the oligonucleotide molecules of the two different binding molecules are different (e.g. have different sequences). The oligonucleotide molecule may be covalently linked to the binding molecules that bind to the nucleic acid binding molecules.

Preferably, the two different binding molecules bind to a different site and/or with a different affinity to the nucleic acid binding molecules.

The oligonucleotide molecule may allow hybridisation of further oligonucleotide molecules to form a circular amplification template.

The binding molecules that bind to the nucleic acid binding molecules may be attached to the oligonucleotide molecule directly or indirectly. In an embodiment in which the binding molecules are attached to the oligonucleotide molecule indirectly, the method may comprise an additional step of adding further binding molecules that bind to the binding molecules (which bind to the nucleic acid binding molecule) and which are attached to the oligonucleotide molecules. Thus, the indirect attachment is via further binding molecules that bind to the two different binding molecules. Thus, the method may comprise the steps:
a) attaching a cell from a suspension of cells to a hydrophilic solid support;
b) adding nucleic acid binding molecules to the cell attached to the solid support so that the nucleic acid binding molecules bind to the nucleic acid end(s) in the cell;
c) adding two different binding molecules that bind to the nucleic acid binding molecules bound to the nucleic acid end(s);
d) adding further binding molecules that bind to the two different binding molecules which are bound to the nucleic acid binding molecules, wherein the further binding molecules are attached to an oligonucleotide molecule;
e) adding further oligonucleotide molecules which hybridise with the oligonucleotide molecules attached to the further binding molecules to form a circular amplification template;
f) performing amplification to produce an amplification product;
g) detecting the amplification product to detect the nucleic acid end(s) in the cell.

The further binding molecules may comprise an antibody or a fragment thereof, a streptavidin molecule, an avidin molecule, a biotin molecule, a protein a peptide, a nucleic acid, an azide, or a polymer. The further binding molecules may be at least two different further binding molecules (e.g. two different antibodies, one of each specific for one of the two different molecules bound to the nucleic acid binding molecules). For example, the binding molecules that bind to the nucleic acid binding molecule may be primary antibodies or fragments thereof and the further binding molecules may be secondary antibodies or fragments thereof that specifically target the primary antibodies. For example, the binding molecules that bind to the nucleic acid binding molecule may be mouse and rabbit primary antibodies, and the further binding molecules may be anti-mouse and anti-rabbit secondary antibodies. The further binding molecules may be directly attached (e.g. conjugated) to an oligonucleotide molecule.

The further oligonucleotide molecules may have the same sequence or at least two different sequences. If the further oligonucleotide molecules have the same sequence, they may hybridise with two oligonucleotide molecules attached to the binding molecules that bind to the nucleic acid binding molecules and form a circular template. If the further oligonucleotide molecules have two different sequences, each of the further oligonucleotide molecules hybridises to one of the oligonucleotide molecules, followed by a step of ligation of the two further oligonucleotide molecules to form a circular template. Thus, the method may further comprise a step of ligation of two further oligonucleotide molecules.

In the step of performing amplification to produce an amplification product, the amplification may be a rolling circle amplification.

The step of detecting the amplification product may be performed using microscopy, methods of automated analysis of high cell numbers, spectroscopy, fluorescence microscopy (wide field, confocal, multifocal, super-resolution, microscopy with catapulting, laser scanning, high throughput, high content), fluorimetry, transmitted light optical microscopy for absorption detection, flow cytometry, cell sorting (FACS), and/or mass spectrometry. The step of detecting the amplification product may include adding a detection molecule, for example, a labelled molecule capable of recognizing and binding to the amplification product. For example, the labelled molecule may bind to the further oligonucleotide molecule sequence. The labelled molecule may comprise a nucleic acid. The labelled molecule may comprise a barcode or a tag.

Preferably, in the methods described herein, the detection is performed *in situ.*

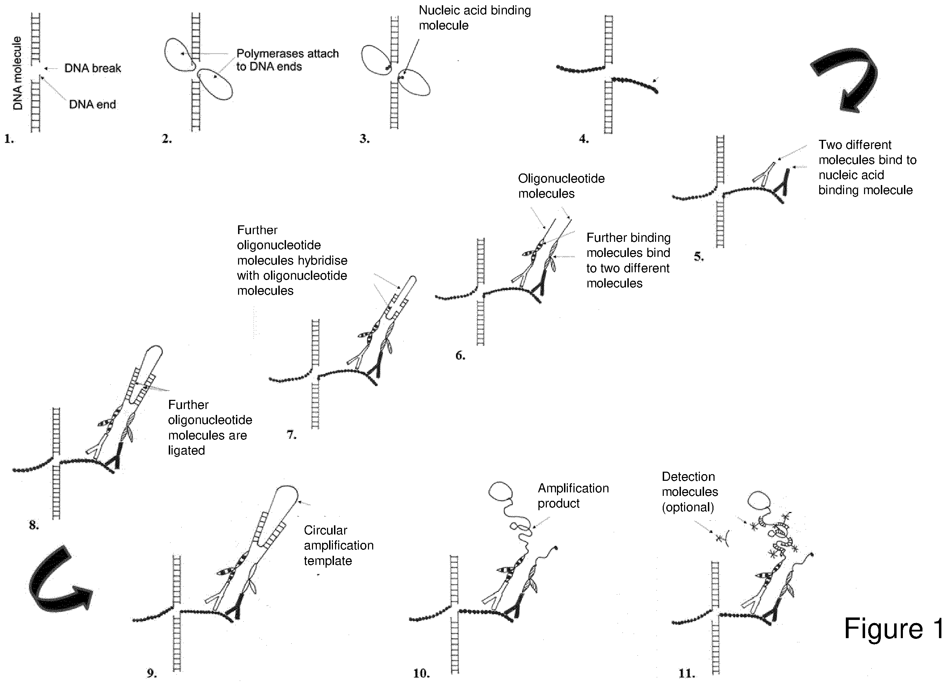

Figure 1 shows an exemplary method which may be performed once the cell(s) are attached to the solid support. 1. A DNA break is formed in a cell. This scheme represents any type of a single- or double-strand DNA break. 2. An enzyme (e.g. polymerase) recognizes the DNA end(s). 3. Nucleic acid binding molecules bind to the DNA end(s). 4. A chain of nucleic acid binding molecules is formed. 5. Two different binding molecules bind to the nucleic acid binding molecules bound to the DNA end(s). 6. Further binding molecules which are attached to oligonucleotide molecules are bound to the two different binding molecules bound to the nucleic acid binding molecules. 7. Further oligonucleotide molecules hybridise with oligonucleotide molecules attached to the further binding molecules. 8. Enzyme ligase ligates the ends of the hybridised further oligonucleotide molecules, thus forming a circular template for rolling circle amplification. 9. A product of ligation, a circular amplification template is ready for the rolling circle amplification reaction. 10. An amplification product is generated - a long oligonucleotide attached to one of the further binding molecules. 11. Detection molecules (e.g. fluorescently labelled oligonucleotides) hybridise with the amplification product, thus marking the presence and position of DNA breaks.

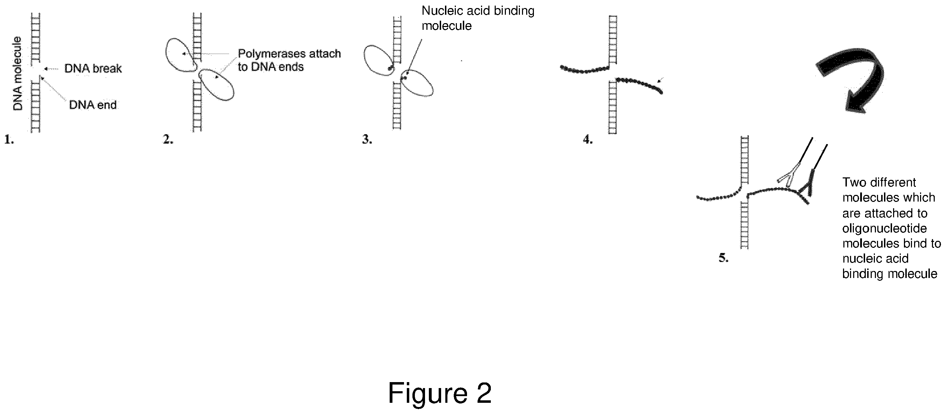

In Figure 2, steps 1-5 of exemplary method which may be performed once the cell(s) are attached to the solid support are shown. Steps 1-4 are the same as in Figure 1. In step 5, the two different binding molecules are directly attached to the oligonucleotide molecules, thus, further binding molecules are not required. Step 5 of this method is followed by step 7 of method of Figure 1. Thus, step 6 of method of Figure 1 is omitted in this example.

The methods described herein may comprise one or more further steps after the step of attaching the cell to the hydrophilic solid support (and, optionally, before the step of adding nucleic acid binding molecules to the cell attached to the solid support). The method may comprise a step of permeabilization of the cell. The method may comprise a step of increasing accessibility of nucleic acid end(s). The method may comprise a step of blocking nonspecific binding site(s) for at least one of the molecules used in the method. The step of permeabilization of the cell may include chemical and/or physical procedures which compromise the integrity of the cell in order to allow or facilitate access of the molecules used in the method to the nucleic acid in the cell. The step of increasing accessibility of nucleic acid end(s) may comprise removing steric hindrance and providing sufficient space to enable access of the molecules used in the methods to the nucleic acid end(s) in the cell.

The step of blocking nonspecific binding site(s) for at least one of the molecules used in the method may comprise blocking of all nonspecific binding sites of the molecules used in the method. The term "blocking" means a procedure leading to prevention of binding of critical molecules used in the assay, for instance, antibodies, to molecular targets other than the ones for which the selected molecules exhibit affinity useful in the assay. A typical example of blocking is a procedure used in antibody detection (immunodetection) of a molecule of interest in biological material. The antibody is directed against a well-defined antigen but can also bind to various cell components via weak chemical interactions. This nonspecific binding is minimized by prior addition of blocking agents (such as albumin) that occupy such binding sites.

The methods described herein may comprise one or more steps of washing. For example, the solid support may be washed after the step of attaching the cell to remove unattached cells and/or other unwanted material. The step of washing may be performed after the step of adding nucleic acid binding molecules. The step of washing may be performed after the step of adding two different binding molecules that bind to the nucleic acid binding molecules. The step of washing may be performed after the step of adding further oligonucleotide molecules. The step of washing may be performed after the step of performing amplification. The step of washing removes unattached or unbound material from the solid support which may produce a clear detection signal and/or less background noise.

The method described herein enables direct detection and visualization of nucleic acid end(s). The method described herein enables marking the presence and position of a single nucleic acid strand break. Thus, the method is particularly useful in detecting nucleic acid damage which has broad utility. For example, the method may be used for assessing the quality of DNA in the subject. The method may also be used to study the mechanism of DNA damage induction, sending and repair. In the context of circulating tumour cells, the method may be particularly useful in assessing effectiveness of a therapeutic agent.

Thus, in one aspect, the invention provides a method for assessing effectiveness of a therapeutic agent, the method comprising the steps:
a) performing the method for detection of nucleic acid end(s) as described herein on a suspension of circulating tumour cells obtained from a subject before the administration of the therapeutic agent,
b) performing the method for detection of nucleic acid end(s) as described herein on a suspension of circulating tumour cells obtained from the subject after the administration of the therapeutic agent,
c) comparing the amount, and optionally the cellular location, of the nucleic acid end(s) detected in steps a) and b), wherein the therapeutic agent is effective if the amount of the nucleic acid end(s) is greater in step b) than step a),
wherein steps a) and b) can be performed in any order.

The subject may be an animal, preferably the subject is human.

Preferably, the nucleic acid end(s) are DNA end(s).

The therapeutic agent may be a cancer drug or drug candidate. The method may be used to assess therapeutic effectiveness of a drug candidate.

The suspension of circulating tumour cells may be obtained from the subject at least 1 hour, 6 hours, 12 hours, 18 hours, 24 hours, 2 days, 3 days, 4 days, 5 days, 6 days, 1 week, 2 weeks, 3 weeks or 4 weeks after the administration of the therapeutic agent. The method may include performing the method for detection of nucleic acid end(s) at multiple time points after the administration of the therapeutic agent. This approach can assess a long-term effect of a therapeutic agent.

The method described herein may also be useful for assessing nucleic acid damage caused by an agent (e.g. a chemical or an environmental agent).

Thus, in an aspect, the invention provides a method for assessing nucleic acid damage caused by an agent, the method comprising the steps:
a) performing the method for detection of nucleic acid end(s) as described herein a suspension of cells obtained from a subject before administration of or exposure to an agent,
b) performing the method for detection of nucleic acid end(s) as described herein on a suspension of cells obtained from the subject after the administration of or exposure to the agent,
c) comparing the amount, and optionally the cellular location, of the nucleic acid end(s) detected in steps a) and b), wherein the agent causes nucleic acid damage if the amount of the nucleic acid end(s) is greater in step b) than step a),
wherein steps a) and b) can be performed in any order.

The nucleic acid damage may be a single strand nucleic acid break or a double strand nucleic acid break. Preferably, the nucleic acid end(s) are DNA end(s). Thus, the nucleic acid damage maybe a single stranded DNA break or a double-stranded DNA break.

The agent may be a therapeutic agent, a chemical agent or an environmental agent.

In the context of a therapeutic agent, the method described herein is able to detected nucleic acid damage caused by the therapeutic agent which may have a side effects. For example, the therapeutic agent may show therapeutic effect in respect of a specific disease or disorder and, at the same time, may cause nucleic acid damage in certain cell types. Thus, the method described herein is able to identify whether the therapeutic agent causes nucleic acid (e.g. DNA) damage.

The chemical agent may be a toxin, an ink, an adhesive, paint, oil, a lubricant, a hair dye, a laboratory reagent, a welding fume, a hazardous medicinal product or a cleaning fluid.

The environmental agent may be a pollutant, a bacterium (or a part thereof), a virus (or a part thereof) or a radioactive substance. The pollutant may be an air pollutant (e.g. a particulate matter, ozone, nitrogen dioxide, carbon monoxide, or sulphur dioxide), a water pollutant (e.g. bacteria, viruses, parasites, fertilisers, pesticides, pharmaceutical products, nitrates, phosphates, plastics, or faecal waste) or a soil pollutant (e.g. pesticides, petroleum products, radon, asbestos, lead, chromated copper arsenate or creosote).

The term "administration" as used herein refers to any type of administration of an agent to the subject. For example, the agent may be administered to the subject by any acceptable route of administration including, but not limited to, oral, topical (including transdermal) and parenteral modes of administration.

The term "exposure to" an agent as used herein is intended to encompass any type of exposure. For example, the subject my be exposed to the agent by inhaling the agent from the air, consuming the agent, absorbing the agent through the skin, eye or a membrane in the subject's body (e.g. nasal membrane or vaginal membrane), or by fluid communication with the agent (e.g. in case of a wound).

The suspension of cells may be obtained from the subject at least 1 hour, 6 hours, 12 hours, 18 hours, 24 hours, 2 days, 3 days, 4 days, 5 days, 6 days, 1 week, 2 weeks, 3 weeks, 4 weeks, 2 months, 3 months, 6 months, or 1 year after the administration of or the exposure to the agent. The method may include performing the method for detection of nucleic acid end(s) at multiple time points after the administration of or the exposure to the agent. This approach can assess a long-term effect of the agent on the health status of the subject.

The methods described herein may be performed using the kits described herein. The skilled person would understand that all molecules, reagents and approaches described in the context of the methods are equally applicable to the kit aspects of the invention. Nonetheless, certain preferred embodiments are also highlighted in the context of the kit aspects of the invention.

Thus, the invention provides a kit for detection of nucleic acid end(s) in a cell, the kit comprising:
a) a hydrophilic solid support;
b) nucleic acid binding molecules;
c) binding molecules that bind to the nucleic acid binding molecules;
d) further oligonucleotide molecules; and
e) reagents for performing amplification.

Preferably, the hydrophilic solid support is a positively charged solid support. For example, the hydrophilic solid support may be TOMO^{®} slide or a poly-L-lysine coated slide.

The nucleic acid binding molecules may comprise halogenated nucleotide or nucleoside molecules, DNA precursor analogues, and/or biotinylated nucleotide molecules. The kit may comprise at least one type of nucleic acid binding molecules. The kit may comprise at least two, at least three or at least four types of nucleic acid binding molecules.

The binding molecules that bind to the nucleic acid binding molecules may be antibodies or fragments thereof, streptavidin molecules, avidin molecules, streptavidin analogues, biotin molecules, peptides, proteins, nucleic acids, azides, or polymers.

Preferably, the binding molecules that bind to the nucleic acid binding molecules are two different molecules. Thus, the kit for detection of nucleic acid end(s) in a cell may comprise:
a) a hydrophilic solid support;
b) nucleic acid binding molecules;
c) two different binding molecules that bind to the nucleic acid binding molecules;
d) further oligonucleotide molecules; and
e) reagents for performing amplification.

In embodiments in which the binding molecules are two different binding molecules, the binding molecules may be of the same time or of a different type. For example, the two different binding molecules may be:
(i) different antibodies or fragments thereof, such as a monoclonal antibody (or a fragment thereof) and a polyclonal antibody (or a fragment thereof);
(ii) different antibodies or fragments thereof, such as a monoclonal antibody (or a fragment thereof) specific to a first nucleic acid binding molecule used in the method, and a monoclonal antibody (or a fragment thereof) specific to a second nucleic acid binding molecule used in the method;
(iii) an antibody or a fragment thereof and a streptavidin,
(iv) an antibody or a fragment thereof and an avidin,
(v) an antibody or a fragment thereof and a protein or peptide; or
(vi) an antibody or a fragment thereof and a biotin molecule.

Preferably, the two different binding molecules bind to a different site and/or with different affinity to the nucleic acid binding molecules.

In one embodiment, the binding molecules may be attached to an oligonucleotide molecule. If two different binding molecules are used, each one of the two different binding molecules is attached to an oligonucleotide molecule.

In another embodiment, the kit may further comprise further binding molecules that are attached to an oligonucleotide molecule. The further binding molecules may be of the same type or of a different type. Preferably, the further binding molecules are of a different type. For example, the further binding molecules may be two (different) antibodies (or fragments thereof). The further binding molecules may be specific and/or selective for binding to the binding molecules bound to the nucleic acid binding molecules.

The further oligonucleotide molecules are, preferably, able to hybridize to the oligonucleotide molecules attached to the binding molecules or further binding molecules present in the kit. The further oligonucleotide molecules may comprise the same or different type of oligonucleotides (e.g. have the same or different sequences). Preferably, the further oligonucleotide molecules comprise at least two types of oligonucleotides (i.e. at least two oligonucleotide sequences). The further binding molecules may be ligated to form a circular template.

The reagents for performing amplification may comprise a polymerase (e.g. a phi29 polymerase), a dNTP mix, a primer (e.g. a hexamer primer), and/or a nuclease-free water.

The kit may further comprise reagents for fixing the cell. For example, the reagent for fixing the cell may be 70%(v/v) ethanol.

The kit may further comprise a polymerase, and/or a ligase. The kit may comprise DNA polymerase I, TdT, Klenow fragment, Phu polymerase, Taq polymerase, T4 DNA Polymerase, T7 DNA Polymerase, T4 Polynucleotide Kinase, and/or RNA polymerase.

The invention also provides the kit described herein for use in the methods described herein.

The invention also provides a hydrophilic solid support for use in the methods and kits described herein. Preferably, the hydrophilic solid support is a positively charged solid support.

The foregoing detailed description has been provided by way of explanation and illustration, and is not intended to limit the scope of the appended claims. Many variations in the presently preferred embodiments illustrated herein will be apparent to one of ordinary skill in the art, and remain within the scope of the appended claims and their equivalents.

The invention is further disclosed in the following clauses:
1. A method for detection of nucleic acid end(s) in a cell, wherein the method comprises the steps of:
   a) attaching a cell from a suspension of cells to a hydrophilic solid support under conditions such that the suspension of cells does not dry out;
   b) adding nucleic acid binding molecules to the cell attached to the solid support under conditions such that the nucleic acid binding molecules bind to the nucleic acid end(s) in the cell;
   c) adding two different binding molecules that bind to the nucleic acid binding molecules bound to the nucleic acid end(s), wherein each of the two different binding molecules is attached to an oligonucleotide molecule;
   d) adding further oligonucleotide molecules which hybridise with the oligonucleotide molecules attached to the two different molecules to form a circular amplification template;
   e) performing amplification to produce an amplification product;
   f) detecting the amplification product to detect the nucleic acid end(s) in the cell.
2. The method of clause 1, wherein the nucleic acid end(s) are DNA end(s), RNA end(s), or DNA:RNA hybrid end(s) preferably DNA end(s).
3. The method of clause 1 or clause 2, wherein the nucleic acid end(s) are single-stranded end(s) or double-stranded end(s).
4. The method of any one of clauses 1-3, wherein the nucleic acid end(s) is a single nucleic acid end.
5. The method of any one of clauses 1-4, wherein the cell is an adherent cell or a suspension cell.
6. The method of any one of clauses 1-5, wherein the cell is a blood cell or a tumour cell.
7. The method of any one of clauses 1-6, wherein the cell is a circulating tumour cell.
8. The method of any one of clauses 1-7, wherein the step of attaching the cell is performed at a temperature of between 10 °C to 40 °C, or 15 °C to 37 °C.
9. The method of any one of clauses 1-8, wherein the step of attaching the cell is performed for between 30 min to 4 hrs, or 1 hr to 3 hrs, preferably 2 hrs.
10. The method of any one of clauses 1-9, wherein the step of attaching the cell is performed in a humidity chamber.
11. The method of any one of clauses 1-10, wherein the suspension of cells has a density of between 0.1 × 10⁶ to 4 × 10⁶ cells/ml, preferably 1.5 x 10⁶ to 2 x 10⁶ cells/ml.
12. The method of any one of clauses 1-11, wherein the hydrophilic solid support is a positively charged slide, such as a TOMO^{®} slide or a poly-L-lysine coated slide, preferably the TOMO^{®} slide.
13. The method of any one of clauses 1-12, wherein the nucleic acid binding molecules are halogenated nucleotide or nucleoside molecules, DNA precursor analogues, and/or biotinylated nucleotide molecules.
14. The method of any one of clauses 1-13, wherein the nucleic acid binding molecules bind to the nucleic acid end(s) by catalytic or noncatalytic means.
15. The method of any one of clauses 1-14, wherein the nucleic acid binding molecules are of the same type or of at least two different types.
16. The method of any one of clauses 1-15, wherein each of the two different binding molecules is attached to the oligonucleotide molecule directly or indirectly.
17. The method of clause 16, wherein the indirect attachment is via further binding molecules that bind to the two different binding molecules.
18. The method of clause 16, wherein the indirect attachment is via a step of addition of further binding molecules that bind to the two different binding molecules.
19. The method of any one of clauses 1-18, wherein the two different binding molecules are:
   (i) different antibodies or fragments thereof,
   (ii) an antibody or a fragment thereof and a streptavidin,
   (iii) an antibody or a fragment thereof and an avidin, or
   (iv) an antibody or a fragment thereof and a biotin molecule.
20. The method of any one of clauses 1-19, wherein the oligonucleotide molecule attached to the first of the two different binding molecules is the same or different as the oligonucleotide molecule attached to the second of the two different binding molecules.
21. The method of any one of clauses 1-20, wherein the further oligonucleotide molecules are identical or comprise at least two different oligonucleotides.
22. The method of any one of clauses 1-21, wherein amplification is a rolling circle amplification.
23. The method of any one of clauses 1-22, wherein the cell is fixed before or after the step of attaching the cell from the suspension of cells.
24. The method of clause 23, wherein fixation is performed in 70%(v/v) ethanol.
25. The method of clause 23 or clause 24, wherein fixation is performed for between 1 h to 24 hrs, 2 hrs to 12 hrs, or 3 hrs to 6 hrs, preferably 4 hrs.
26. The method of any one of clauses 23-25, wherein fixation is performed at a temperature of between -80 °C to 20°C, -40 °C to 10°C, or -20 °C to 4°C.
27. The method of any one of clauses 1-26, wherein the suspension of cells is removed after the step of attaching and before the step of adding nucleic acid binding molecules to remove unattached cells.
28. The method of any one of clauses 1-27, wherein the cell is stored at a temperature of between -80 °C to -4°C, or -30 °C to -15°C, after the step of attaching and before the step of adding nucleic acid binding molecules, preferably, wherein the cell is stored at a temperature of - 20°C.
29. The method of clause 28, wherein the cell is stored for at least 2 hrs, at least 3 hrs, at least 4 hrs, at least 5 hrs, or at least 6 hrs, preferably at least 4 hrs.
30. A method for detection of nucleic acid end(s) in a circulating tumour cell, wherein the method comprises the steps of:
   a) attaching a circulating tumour cell from a suspension of circulating tumour cells to a hydrophilic solid support under conditions such that the suspension of circulating tumour cells does not dry out;
   b) adding nucleic acid binding molecules to the cell attached to the solid support under conditions such that the nucleic acid binding molecules bind to the nucleic acid end(s) in the cell;
   c) adding two different binding molecules that bind to the nucleic acid binding molecules bound to the nucleic acid end(s), wherein each of the two different binding molecules is attached to an oligonucleotide molecule;
   d) adding further oligonucleotide molecules which hybridise with the oligonucleotide molecules attached to the two different binding molecules to form a circular amplification template;
   e) performing amplification to produce an amplification product;
   f) detecting the amplification product to detect the nucleic acid end(s) in the cell.
31. A method for detection of nucleic acid end(s) in a cell, wherein the method comprises the steps of:
   a) attaching a cell from a suspension of cells to a hydrophilic solid support under conditions such that the suspension of cells does not dry out;
   b) removing the suspension of cells to remove unattached cells;
   c) fixing the cell attached to the solid support;
   d) adding nucleic acid binding molecules to the cell attached to the solid support under conditions such that the nucleic acid binding molecules bind to the nucleic acid end(s) in the cell;
   e) adding two different binding molecules that bind to the nucleic acid binding molecules bound to the nucleic acid end(s), wherein each of the two different binding molecules is attached to an oligonucleotide molecule;
   f) adding further oligonucleotide molecules which hybridise with the oligonucleotide molecules attached to the two different molecules to form a circular amplification template;
   g) performing amplification to produce an amplification product;
   h) detecting the amplification product to detect the nucleic acid end(s) in the cell.
32. A method for detection of nucleic acid end(s) in a cell, wherein the method comprises the steps of:
   a) attaching a cell from a suspension of cells to a hydrophilic solid support under conditions such that the suspension of cells does not dry out;
   b) removing the suspension of cells to remove unattached cells;
   c) fixing the cell attached to the solid support;
   d) storing the cell at a temperature of between -30 °C to -15°C for at least 4 hrs;
   e) adding nucleic acid binding molecules to the cell attached to the solid support under conditions such that the nucleic acid binding molecules bind to the nucleic acid end(s) in the cell;
   f) adding two different binding molecules that bind to the nucleic acid binding molecules bound to the nucleic acid end(s), wherein each of the two different binding molecules is attached to an oligonucleotide molecule;
   g) adding further oligonucleotide molecules which hybridise with the oligonucleotide molecules attached to the two different binding molecules to form a circular amplification template;
   h) performing amplification to produce an amplification product;
   i) detecting the amplification product to detect the nucleic acid end(s) in the cell.
33. A kit for detection of nucleic acid end(s) in a cell, the kit comprising:
   a) a hydrophilic solid support;
   b) nucleic acid binding molecules;
   c) two different binding molecules that bind to the nucleic acid binding molecules;
   d) further oligonucleotide molecules; and
   e) reagents for performing amplification.
34. The kit of clause 33, wherein the two different binding molecules are attached to an oligonucleotide molecule.
35. The kit of clause 33 or clause 34, wherein the kit further comprises further binding molecules that are attached to an oligonucleotide molecule.
36. The kit of any one of clauses 33-35 for use in the method of any one of clauses 1-32.
37. A method for assessing effectiveness of a therapeutic agent, the method comprising the steps:
   a) performing the method of any one of clauses 1-32 on a suspension of circulating tumour cells obtained from a subject before the administration of the therapeutic agent,
   b) performing the method of any one of clauses 1-32 on a suspension of circulating tumour cells obtained from the subject after the administration of the therapeutic agent,
   c) comparing the amount, and optionally the cellular location, of the nucleic acid end(s) detected in steps a) and b), wherein the therapeutic agent is effective if the amount of the nucleic acid end(s) is greater in step b) than step a),
   wherein steps a) and b) can be performed in any order.
38. A method for assessing nucleic acid damage caused by an agent, the method comprising the steps:
   a) performing the method of any one of clauses 1-32 on a suspension of cells obtained from a subject before administration of or exposure to an agent,
   b) performing the method of any one of clauses 1-32 on a suspension of cells obtained from the subject after the administration of or exposure to the agent,
   c) comparing the amount, and optionally the cellular location, of the nucleic acid end(s) detected in steps a) and b), wherein the agent causes nucleic acid damage if the amount of the nucleic acid end(s) is greater in step b) than step a),
   wherein steps a) and b) can be performed in any order.

These and other aspects of the invention will now be described with reference to the accompanying Figures, in which:

### DESCRIPTION OF THE FIGURES

Figure 1 shows an exemplary detection method, in which the binding molecules bound to the nucleic acid binding molecules are bound by further binding molecules that carry the oligonucleotides.
Figure 2 shows the first five steps of an exemplary detection method of Figure 1. In this method, the binding molecules that are bound to the nucleic acid binding molecules are also attached to the oligonucleotide molecules. Thus, there is no need to add further binding molecules. After step 5, the method continues as in step 7 onwards of Figure 1.

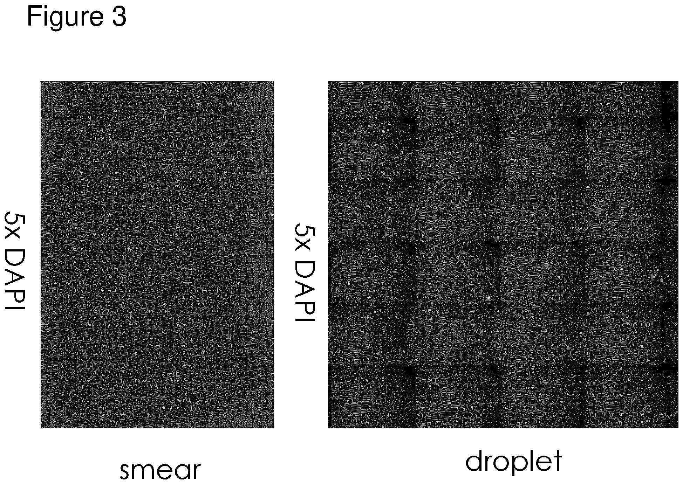

Figure 3 illustrates the results for the attachment of cells to TOMO slides using the dry attachment method (i.e. "smear") and the wet attachment method (i.e. "droplet").

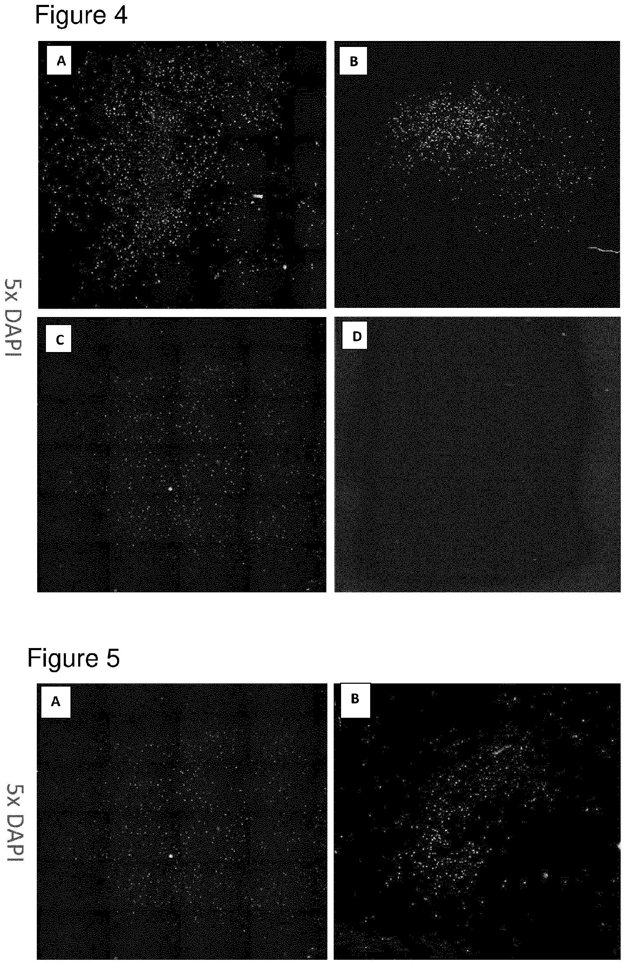

Figure 4 shows the results for the detection of nucleic acid end(s) using the methods described herein for samples prepared on TOMO slides. (A) "fresh" cells wet attachment to TOMO slide (B) cells from 48h bank wet attachment to TOMO slide (C) cells from 4 weeks bank wet attachment to TOMO slide (D) "fresh" cells smear on TOMO slide.
Figure 5 shows the results for the detection of nucleic acid end(s) using the methods described herein for samples prepared on TOMO slides. (A) cells from 4 weeks bank wet attachment to TOMO slide (B) cells attached in wet procedure to TOMO slide and stored in 70% ethanol (v/v) at -20°C for 3 weeks.

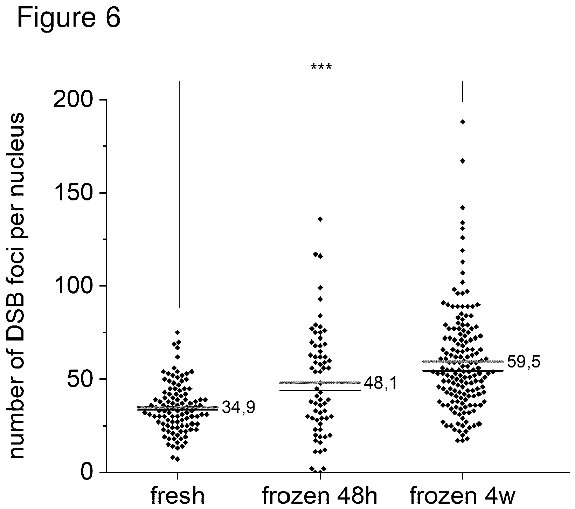

Figure 6 shows the number of double stranded break foci per nucleus detected on "fresh" cells attached by the method described herein, cells frozen for 48 hrs and cells frozen for 4 weeks.

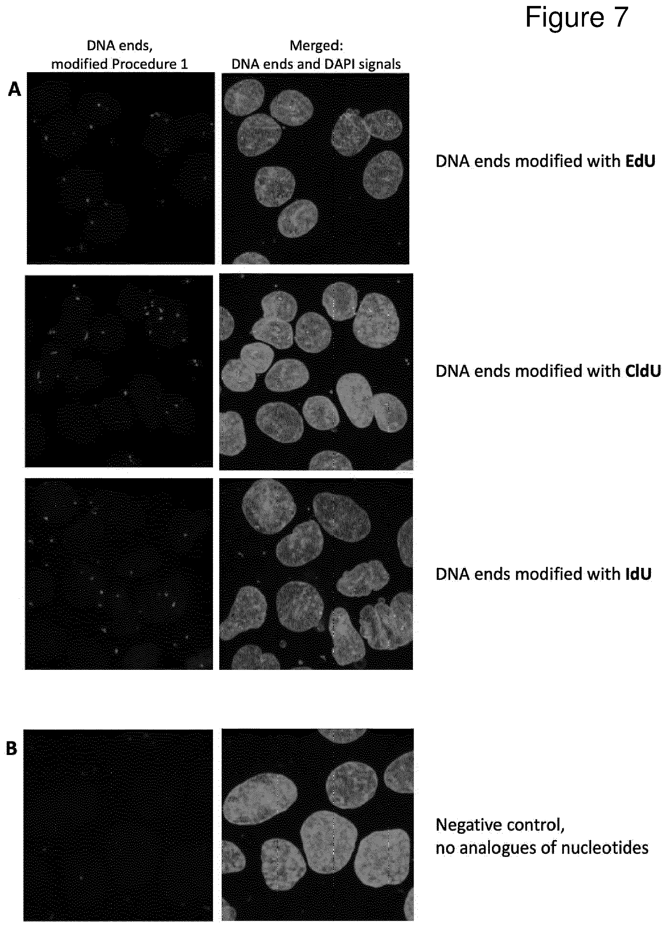

Figure 7 illustrates results for binding of various nucleic acid binding molecules to the DNA end(s). (A) Fluorescence confocal images of cell nuclei with DNA ends visualised by the methods described herein (Procedure 1, in which BrdU molecules were replaced with EdU, CldU or IdU molecules). (B) Fluorescence confocal images of a negative control sample that was processed following the protocol described in Procedure 1 in which the analogues of nucleosides were replaced with DMSO.

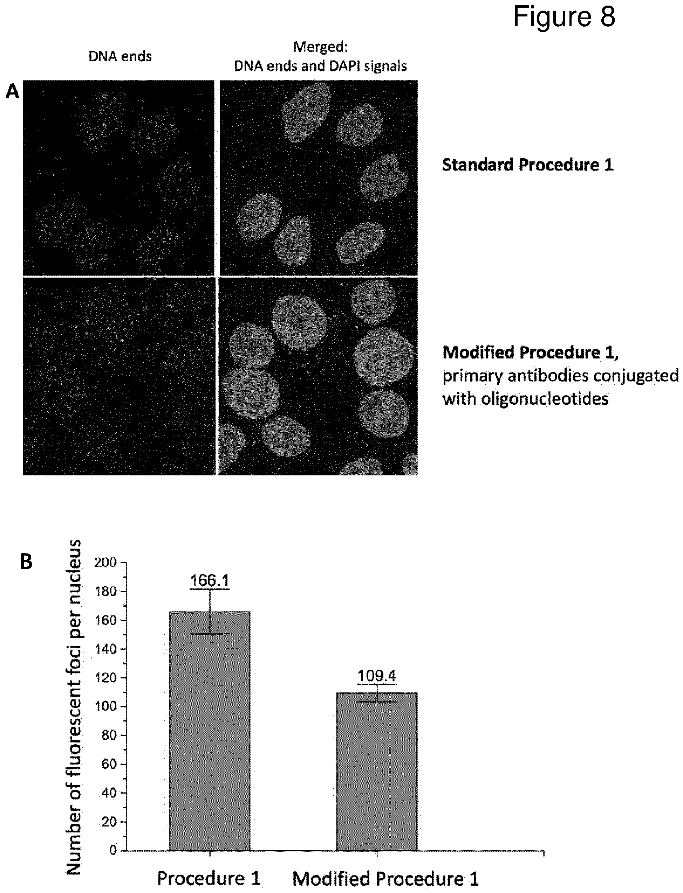

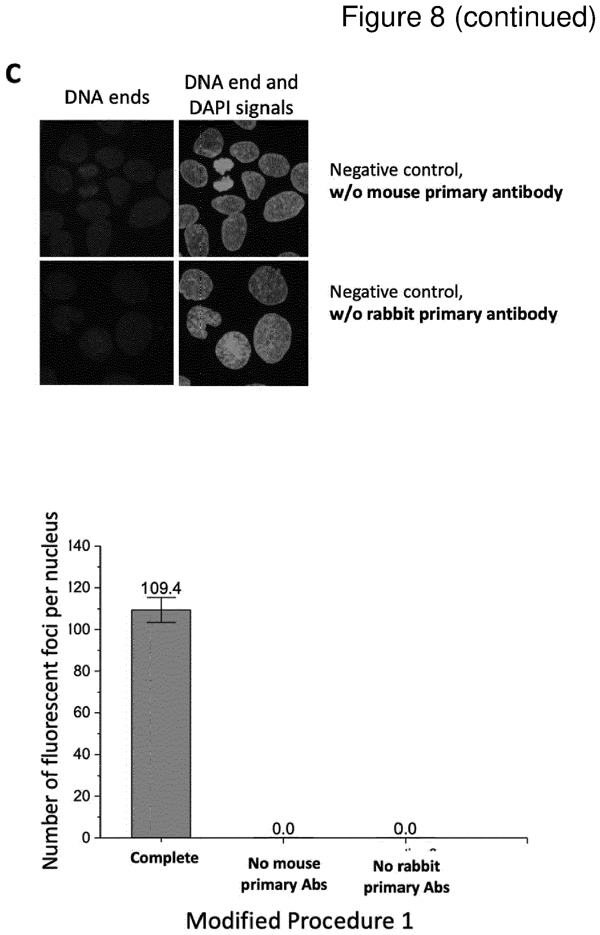

Figure 8 illustrates detection of free DNA ends in untreated U2OS cells using the method described herein (Procedure 1, in which further binding molecules (secondary antibodies) were omitted and the primary antibodies were conjugated with oligonucleotides). (A) Fluorescence confocal images of cell nuclei with DNA ends labelled following two protocols: standard Procedure 1 and modified Procedure 1, in which further binding molecules (secondary antibodies) were omitted and the primary antibodies were conjugated with oligonucleotides. (B) Results of quantitative image analysis - comparison of the average number of fluorescent foci detected per nucleus between the sample labelled using Procedure 1 and the one labelled following modified Procedure 1. (C) Fluorescence confocal images and results of quantitative analysis of images presenting negative control samples that were processed following the modified Procedure 1, in which one of the primary antibodies was omitted. In each such case no foci were detected.

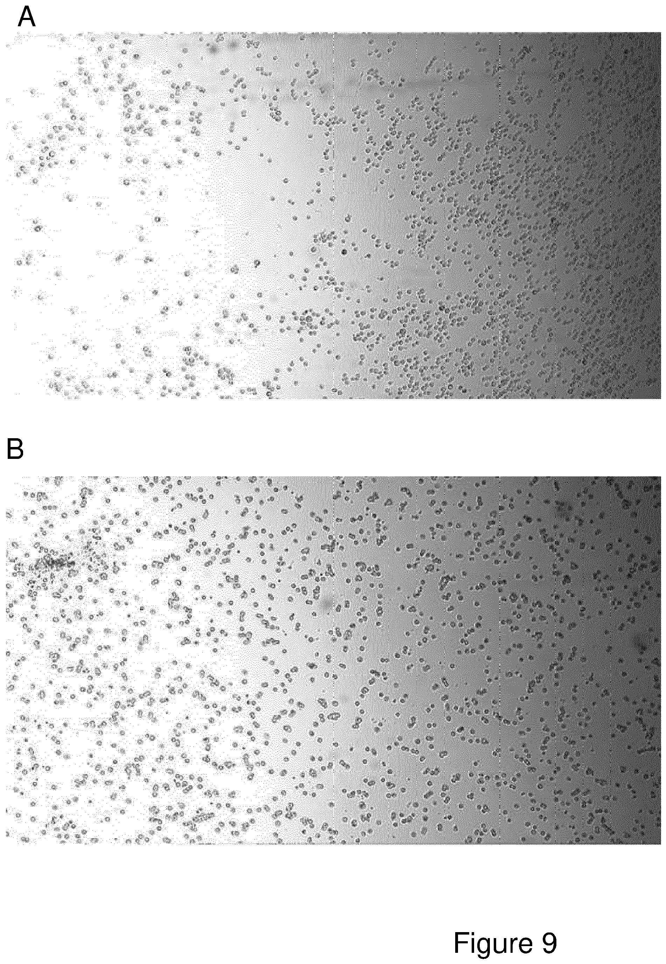

Figure 9 illustrates the distribution of LNCaP cells on a solid support. Figure 9A shows distribution of cells using a protocol in which LNCaP cells were first attached to the solid support and then fixed. Figure 9B shows distribution of cells using a protocol in which LNCaP cells were first fixed and then attached to the solid support.

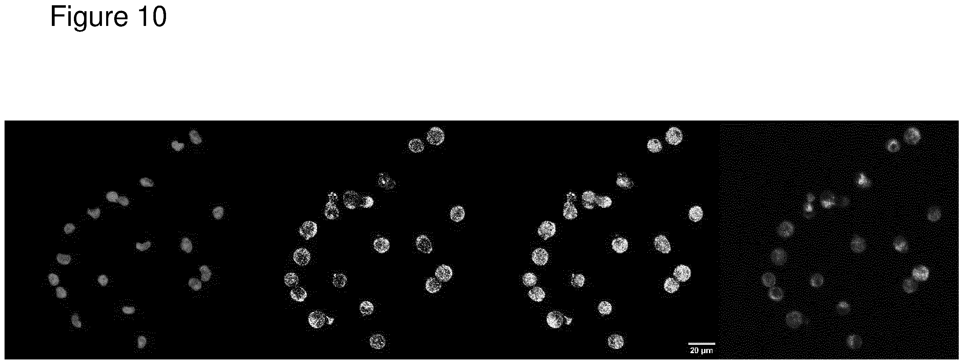

Figure 10 shows an exemplary detection image of nucleic acid end(s) in LNCaP cells using the detection method described herein. LNCaP cells were first attached to the solid support and then fixed. Blue - DAPI (nuclei), Yellow - dSTRIDE (double-strand DNA breaks), Green - pan Cytokeratin.

### EXAMPLES

The present invention is explained more in detail with the aid of the following examples which are not intended to limit the scope of the present invention in any manner.

Examples are described on the basis of general protocols which concern main embodiments presented in the description.

General procedures for nucleic acid end(s) detection - the detection steps are as described in WO2019/035727 A1 (and briefly reproduced below).

### Exemplary Procedure 1

Exemplary procedure for *in situ* DNA damage detection performed using enzyme TdT (terminal deoxynucleotidyl transferase) - detection of single-strand gaps, double-strand blunt-end breaks or double-strand 3'-protruding breaks.

### Procedure:

1. Addition of nucleic acid binding molecules
   E.g. incorporation of unmodified and modified nucleotides (e.g. BrdU) using terminal deoxynucleotidyl transferase:
   a. incubation is performed on a droplet in a humid chamber
   b. samples are rinsed in wash buffers
2. Optional blocking:
   Incubation with 1- 5% BSA solution in PBS (from 1 h to overnight in 4°C, *RT*)*.*
3. Incubation with two different binding molecules (e.g. primary antibodies):
   a. primary antibodies type 1 (e.g. mouse monoclonal against BrdU), preferred dilution 1:100 in 1-5% BSA, 1 h, RT
   b. (optional) wash (PBS 3 × 5 min)
   c. primary antibodies type 2 (e.g. rabbit polyclonal against BrdU), preferred dilution 1:100 in 1-5% BSA, 1 h, RT
   d. (optional) wash (PBS 3 × 5 min)
4. Amplification:
   a. Optional (required if the two different molecules are not attached to oligonucleotide molecules directly): incubation with PLA Probes diluted in 1% BSA (37 °C, 60 min, reaction on a droplet, in a humid chamber) in 40 µl of the reaction mixture per one coverslip:
      - 8 µl of PLA Probe MINUS stock (e.g. anti-mouse)
      - 8 µl of PLA Probe PLUS stock (e.g. anti-rabbit)
      - 24 µl of 1% BSA
   b. (optional) wash samples with Wash Buffer A (2 x 5 min)
   c. ligation - incubation (37°*C*, 30 min, reaction on a droplet, in a humid chamber) in 40 µl of the reaction mixture:
      - 8 µl of 5x Ligation stock (Sigma, Duolink, DUO82009),
      - 31 µl of ultrapure, RNAse/DNAse free, distilled water,
      - 1 µl of Ligase (1 U/µl) (Sigma, Duolink, DUO82027 or DUO82029)
   d. (optional) wash samples with Wash Buffer A (2 x 2 min)
   e. amplification - incubation (37°*C*, 100 min, reaction performed on a droplet, in a humid chamber) in 40 µl of the reaction mixture:
      - 8 µl of 5x Amplification stock (Sigma, Duolink, DUO82060 or DU082011),
      - 31.5 µl of ultrapure, RNAse/DNAse free, distilled water,
      - 0.5 µl of Polymerase (10 U/µl) (Sigma, Duolink, DUO82028 or DUO82030)
5. Imaging of samples in PBS using a fluorescence microscope.

### Exemplary Procedure 2

Exemplary procedure of DNA damage detection performed using enzyme DNA polymerase I - detection of single-strand nicks, single-strand gaps and double-strand 3' recessed ends.
1. Addition of nucleic acid binding molecules
   E.g. incorporation of unmodified and modified (e.g. biotin-conjugated) nucleotides using DNA polymerase I:
   a. Rinse the samples with distilled water
   b. Incubation (5 - 60 min, RT on a droplet, in a humid chamber) in reaction mixture consisting of:
      - reaction buffer
      - dNTPs (the ratio of modified to unmodified nucleotides varies from 3:1 to 1:3)
      - DNA polymerase I
      - ultrapure, RNAse/DNAse free, distilled water
   c. Reaction termination:
      - incubation with 50 mM TrisHCl (pH 7.48), 2 x 10 min, RT
      - incubation with 0.5 mM TrisHCl , 1 × 1 min RT
2. Optional blocking:
   1- 5% BSA solution in PBS (from 1 h to overnight in 4°C, *RT*)*.*
3. Incubation with two different binding molecules (e.g. primary antibodies):
   a. primary antibodies type 1 (e.g. mouse monoclonal against biotin), preferred dilution 1:100 in 1-5% BSA, 1 h, RT
   b. (optional) wash (PBS 3 × 5 min)
   c. primary antibodies type 2 (e.g. rabbit polyclonal against biotin), preferred dilution 1:100 in 1-5% BSA, 1 h, RT
   d. (optional) wash (PBS 3 × 5 min)
4. Amplification
   a. Optional (required if the two different binding molecules are not attached to oligonucleotide molecules directly): incubation with PLA Probes diluted in 1% BSA (37 °C, 60 min, reaction on a droplet, in a humid chamber) in 40 µl of the reaction mixture per one coverslip:
      - 8 µl of PLA Probe MINUS stock (e.g. anti-mouse)
      - 8 µl of PLA Probe PLUS stock (e.g. anti-rabbit)
      - 24 µl of 1% BSA
   b. (optional) wash samples with Wash Buffer A (2 x 5 min)
   c. ligation - incubation (37°*C*, 30 min, reaction on a droplet, in a humid chamber) in 40 µl of the reaction mixture per one coverslip:
      - 8 µl of 5x Ligation stock (Sigma, Duolink, DUO82009),
      - 31 µl of ultrapure, RNAse/DNAse free, distilled water,
      - 1 µl of Ligase (1 U/µl)(Sigma, Duolink, DUO82027 or DUO82029)
   d. (optional) wash samples with Wash Buffer A (2 x 2 min)
   e. amplification - incubation (37°*C*, 100 min, reaction performed on a droplet, in a humid chamber) in 40 µl of the reaction mixture per one coverslip:
      - 8 µl of 5x Amplification stock (Sigma, Duolink, DUO82060 or DUO82011),
      - 31.5 µl of ultrapure, RNAse/DNAse free, distilled water,
      - 0.5 µl of Polymerase (10 U/µl) (Sigma, Duolink, DUO82028 or DUO82030)
5. Imaging of samples in PBS using a fluorescence microscope.

The detection of nucleic acid end(s) in the Examples relies on the detection method as described in WO2019/035727 A1. Further modifications have been made to the detection method of WO2019/035727 A1 in Examples 2 and 3. Example 1 shows an attachment method of 22RV1 cells to a solid support. Example 4 shows a protocol for attachment of LNCap cells to a solid support.

### Example 1

### Attachment of 22RV1 cells to glass slides and detection of DNA breaks

### Cell culture:

In this experiment 22RV1 cells (obtained from ATCC) were used. Cells were grown at 37°C with 5% CO2 in T75 flasks with RPMI 1640 (Gibco, 11875101) + 10% FBS (Gibco, A4736401) + 1% Penicillin-Streptomycin Solution (Gibco, 15140130) (10000 units each). When cell confluence reached 80%, cells were washed with sterile PBS (Gibco, 20012027, without Ca2+ and Mg2+) and incubated for 2 minutes with 2 mL of 0.25% trypsin solution in EDTA (Gibco, 25200056). Trypsin was inactivated with the culture medium containing FBS and the collected cells were counted in a Burker chamber.

### Banking protocol:

Cell suspension was centrifuged at room temperature (1000 rpm, 5 minutes). Then medium was removed and 1mL of sterile FBS with 10% DMSO (Thermo Scientific, J66650.AD) was added. The mixture was mixed gently and pipetted to cryotube. The cryotube was transferred to -80°C in Nalgene Mr. Frosty Freezing Container for slow freezing (decreasing the temperature approximately 1 °C per minute). After 120 minutes the cryotube was transferred to liquid nitrogen. The mixture was stored for 48 hours, 1 week and 3 weeks in the liquid nitrogen.

### Types of slides used:

1) Glass Slides (Epredia)
2) Poly-L-lysine coated slides (Thermo Scientific)
3) TOMO slides
4) Coverslips 18 µm thickness (Epredia)
5) Coverslips coated with aminosilane* (Epredia)
6) 96-well plate (Greiner)
*(3-Aminopropyl)trimethoxysilane coating

### Attachment procedure:

15,000 to 20,000 of living 22RV1 cells were suspended in around 100 µL of culture medium with supplements.

Prior to wet attachment, an area of 2×2cm was outlined on a glass slide with a hydrophobic pen, and the cells were incubated at the centre of the slide. For wet attachment, at no point cells dried out. For some (where indicated), a humidity chamber was used to avoid drying out. 100 µL of cells suspension were incubated at the centre of the microscopy slide.

Smears were prepared according to the method below.

### Smear preparation:

1. place a drop of well mixed cells (about 100 µL) near the frosted end of a slide;
2. place another slide (or rectangular coverslips) in front of cells suspension drop.
3. Holding the spreader slide at an approximately 30-45° angle, back into the drop of cells suspension and allow the drop to spread along the edge of the spreader slide;
4. push the spreader slide forward (towards the unfrosted end) in a smooth, moderately fast motion. Apply only enough pressure to keep the spreader slide on the glass. The fluid runs along the edge of the slide by capillary action;
5. allow smears to dry for 2 hours at room temperature in a horizontal position.

### Conditions of cell attachment which were assessed:

a. Glass slides, 2 hours at 37°C, wet attachment, humidity chamber
b. poly-L-lysin coated slides, 2 hours at 37°C, wet attachment, humidity chamber
c. TOMO slides, 2 hours at 37°C, wet attachment, humidity chamber
d. Glass slides, 2 hours at 37°C, wet attachment, humidity chamber, cells from 48 hour bank, resuspended in medium with supplementation
e. poly-L-lysin coated slides, 2 hours at 37°C, wet attachment, humidity chamber cells from 48 hour bank, resuspended in medium with supplementation
f. TOMO slides, 2 hours at 37°C, wet attachment, humidity chamber, cells from 48 hour bank, resuspended in medium with supplementation
g. TOMO slides, 2 hours at 37°C, wet attachment, humidity chamber cells from 4 weeks bank, resuspended in medium with supplementation
h. Cover glasses, 2 hours at 37°C, wet attachment
i. Cover glasses, 2 hours at 37°C, wet attachment, cells from 1 week bank resuspended in medium with supplementation
j. Cover glasses coated with aminosilane, 2 hours at 37°C, wet attachment
k. 96-well plate, 2 hours at 37°C, wet attachment
l. 96-well plate, 2 hours at 37°C, wet attachment, cells from 1 week bank resuspended in medium with supplementation
m. Glass slides, 2 hours at room temperature, smear
n. poly-L-lysin coated slides, 2 hours at room temperature, smear
o. TOMO slides, 2 hours at room temperature, smear
p. Glass slides, 2 hours at room temperature, smear, cells from 48 hour bank resuspended in medium with supplementation
q. poly-L-lysin coated slides, 2 hours at room temperature, smear, cells from 48 hour bank resuspended in medium with supplementation
r. TOMO slides, 2 hours at room temperature, smear, cells from 48 hour bank resuspended in medium with supplementation

After incubation, the medium was gently removed. The cells were then fixed in 70% ethanol (v/v) at - 20°C for at least 4 hours.

In c) condition - TOMO slides, 2h at 37°C, wet attachment, humidity chamber higher cell densities were tested - 40k, 60k, 100k.

Glass from c) condition - TOMO slides, 2h at 37°C, wet attachment, humidity chamber was stored in 70% ethanol (v/v) at -20°C for 3 weeks.

Cells were then stained using the method as described in Procedures 1 or 2.

### Results:

Non-satisfactory results were achieved in experiments with glass slides and 96-well plates. Approximately 80% of cells were lost during the first wash after fixation, and the antigen retrieval step resulted in the loss of 100% cells during the detection procedure.

Procedures using TOMO slides wet attachment allowed to achieve high cell confluence after the detection procedure using 15,000 to 20,000 of cells. Use of higher cell densities (40,000, 60,000, 100,000) led to all cells being washed off the glass during the first wash. Non-satisfactory results were achieved in experiments with smears on TOMO slides. The results are shown in Figure 3.

The highest efficiency of cell attachment was achieved for living and frozen cells incubated at 37°C for 2 hours on TOMO slides (wet attachment).

Long-term storage of cells on TOMO slides after immobilization in 70% ethanol (v/v) at -20°C had an effect on cells density after staining. Approximately 40%-50% was lost during the detection procedure. The results are shown in Figure 4.

Cells attached in wet procedure to TOMO slide and stored in 70% ethanol (v/v) at -20°C for 3 weeks was stained using the method described herein. The final density of cells is shown in Figure 5.

Figure 6 shows a number of double stranded breaks foci detected in fresh immobilized cells, in cells frozen for 48 hrs and in cells frozen for 4 weeks.

### Example 2

### Detection of free DNA ends in untreated U2OS cells using the method according to Procedure 1 - feasibility of incorporation of modified nucleotides other than BrdU.

U2OS cells were used. Cells were seeded on 18-mm coverslips (the number of seeded cells was 120 × 10³ per 1 coverslip) and cultured for 2 days in DMEM supplemented with 10% FBS. Subsequently, cells were incubated in 70% ethanol in water for 12 hours at -20°C. EdU, CldU or IdU was linked to free ends of DNA using TdT enzyme using APO-BRDU kit (Phoenix Flow Systems, AU: 1001). After the extension of DNA ends, the samples were incubated with 1% BSA (solution in PBS) for 1 hour at room temperature. Duolink^{®} Detection Reagents Red (Merck, DUO92008) were used in the final step of the amplification reaction according to Procedure 1. The sample in which no analogues of nucleotides were used (they were replaced with DMSO) in the reaction with TdT was treated as a negative control sample. After treatment the samples were imaged and analysed using a Zeiss Cell Discoverer 7 confocal microscope (excitation: 590 nm, emission: 618 nm).

### Results:

Most of the untreated U2OS cells (apart from the negative control sample) subjected to complete Procedure 1 displayed a few (at least one) fluorescent foci, representing free DNA ends at the sites of endogenous double-strand DNA breaks inside the cell nucleus (Figure 7A). In the case of the reference negative control samples, the majority of imaged nuclei did not contain any fluorescent foci at all (Figure 7B).

The numbers of detectable foci were quantified and summarized in Table 1 below. The differences between the average numbers of foci detected in the nuclei in which analogues of nucleotides were incorporated and the counts quantified in control cells are statistically significant (see p-Values in Table 1 below). This study confirms that analogues of nucleotides other than BrdU or biotin-conjugated nucleotides can be effectively used in the presented protocols for detection and labelling of DNA ends.

**Table 1. Quantification of observed fluorescence foci labelling DNA ends detected inside cell nuclei.**

| | CIdU | IdU | EdU | Negative control |
|---|---|---|---|---|
| Number of DSB foci per nucleus (mean) | 4.62 | 5.58 | 3.27 | 0.6 |
| Standard error | ±0.98 | ±1.43 | ±0.84 | ±0.4 |
| | | | | |
| T-test | CIdU - Neg. Ctrl | IdU - Neg. Ctrl | EdU - Neg.Ctrl | |
| p-Value | 0.000392663 | 0.002689789 | 0.004743265 | |

### Example 3

### Detection of free DNA ends in untreated U2OS cells using the method according to modified Procedure

### 1, in which PLA Probes (secondary antibodies) were omitted and the primary antibodies were conjugated with oligonucleotides.

In this experiment U2OS cells were used. Cells were seeded on 18-mm coverslips (the number of seeded cells was 120 × 10³ per 1 coverslip) and cultured for 2 days in DMEM supplemented with 10% FBS. Subsequently, cells were incubated in 70% ethanol in water for 12 hours at -20°C. After incubation in 70% ethanol the cells were treated following the same protocol as the one described in Example 2 with the exception that DNA ends were modified by addition of BrdU molecules. In a subset of samples, the primary antibodies were conjugated to oligonucleotides necessary for the RCA reaction and secondary antibodies (PLA Probes) were omitted in the protocol. Prior to the experiment the primary antibodies were conjugated with oligonucleotides using Duolink^{®} InSitu Probemaker MINUS (Merck, DUO92010) and Duolink^{®} In Situ Probemaker PLUS (Merck, DUO92009). Duolink^{®} Detection Reagents Red (Merck, DUO92008) were used in the final step of the PLA reaction. The samples in which one of the primary antibodies was omitted (either mouse or rabbit) were used as negative control samples. After treatment the samples were imaged and analysed using a Zeiss Cell Discoverer 7 confocal microscope (excitation: 590 nm, emission: 618 nm).

### Results:

The modification of the labelling protocol in which the secondary antibodies (PLA Probes) are omitted results in the occurrence of numerous bright fluorescent foci at the sites of DNA ends which are associated with double-strand DNA breaks in the vast majority of images cell nuclei. The fluorescent confocal images and the graphs presenting the quantification of fluorescent foci detectable inside the nuclei obtained in the samples in which the conjugated primary antibodies were used are comparable with the results obtained in the samples where the standard protocol, according to the Procedure 1, was applied (Figure 8). The characteristics of the foci and their localisation in the case of both protocols are aligned with the biological meaning of the signals and confirm that both procedures can be used for labelling of DNA ends that occur at the sites of DNA breaks or DNA fragments.

### Conclusions:

This experiment confirms that the application of secondary antibodies in Procedure 1 is not essential for the *in situ* detection of DNA ends and the use of primary antibodies conjugated with oligonucleotides used in the amplification reaction is sufficient to obtain correct labelling patterns and meaningful results.

### Example 4

### Protocol for attachment of LNCap cells to the solid support, followed by fixation

1. Culture the cells on a flask. Detach it with trypsine and neutralize with medium.
2. Count the cells in a Burker chamber and place desired number (80-120k cells) in 1.5ml Eppendorf tube.
3. Spin it down and resuspend in approximately 160 ul of PBS.
4. On the center of poly-L-lysine coated slide (Epredia), outline an area of diameter approximately 1.5 cm, using a hydrophobic pen.
5. Place cells in PBS on a poly-L-lysine coated slide (Epredia). Place the cell suspension in the centre of the outlined area on the slide.
6. Incubate the slide(s) for 2 hours in a CO2 incubator at 37C. Place slides in humidity chamber to avoid drying.
7. After the incubation, gently remove the remaining excess of the PBS.
8. Fix the attached cells in ice-cold 70% ethanol - move to -20C freezer for at least 4 hours before taking any further action.

### Protocol for fixing LNCap cells followed by attachment to the solid support.

1. Culture the cells on a flask. Detach it with trypsine and neutralize with medium.
2. Count the cells in a Burker chamber and place desired number (80-120k cells) in 1.5ml Eppendorf tube.
3. Spin it down and resuspend in approximately 160 ul of ice-cold 70% ethanol.
4. Move to -20C freezer for at least 4 hours before taking any further action.
5. On the center of poly-L-lysine coated slide (Epredia), outline an area of diameter approximately 1.5 cm, using a hydrophobic pen.
6. Place the cells in ice-cold 70% ethanol on poly-L-lysine coated slide (Epredia)
7. Incubate the slide in ethanol for 2 hours at room temperature (about 22°C).
8. After the incubation, gently remove the remaining excess of ethanol
9. A. For detection - proceed with staining procedure; B. For storage - incubate the cells in ethanol prior to storage.

The distribution of the cells on solid support is shown in Figure 9. Figure 9A shows distribution of cells using a protocol in which LNCaP cells were first attached to the solid support and then fixed. Figure 9B shows distribution of cells using a protocol in which LNCaP cells were first fixed and then attached to the solid support.

Figure 10 shows an image of LNCaP cells in which nucleic acid end(s) were labelled using the detection method described herein. LNCaP cells were first attached to the solid support and then fixed.

## Claims

1. A method for detection of nucleic acid end(s) in a cell, wherein the method comprises the steps of:
a) attaching a cell from a suspension of cells to a hydrophilic solid support under conditions such that the suspension of cells does not dry out;
b) adding nucleic acid binding molecules to the cell attached to the solid support under conditions such that the nucleic acid binding molecules bind to the nucleic acid end(s) in the cell;
c) adding two different binding molecules that bind to the nucleic acid binding molecules bound to the nucleic acid end(s), wherein each of the two different binding molecules is attached to an oligonucleotide molecule;
d) adding further oligonucleotide molecules which hybridise with the oligonucleotide molecules attached to the two different binding molecules to form a circular amplification template;
e) performing nucleic acid amplification to produce an amplification product; and
f) detecting the amplification product to detect the nucleic acid end(s) in the cell.

2. The method of claim 1, wherein the cell is:
i. an adherent cell or a suspension cell; and/or
ii. a blood cell, a tumour cell, or a circulating tumour cell.

3. The method of claim 1 or claim 2, wherein the step of attaching the cell is performed:
i. at a temperature of between 10 °C to 40 °C, or between 15 °C to 37 °C;
ii. for between 30 min to 4 hrs, or between 1 hr to 3 hrs, preferably 2 hrs; and/or
iii. in a humidity chamber and/or a CO2 incubator.

4. The method of any one of claims 1-3, wherein the hydrophilic solid support is a positively charged solid support, such as a TOMO^{®} slide or a poly-L-lysine coated slide.

5. The method of any one of claims 1-4, wherein each of the two different binding molecules is attached to the oligonucleotide molecule directly or indirectly, optionally wherein
the indirect attachment is via:
i. further binding molecules that bind to the two different molecules; or
ii. a step of addition of further binding molecules that bind to the two different binding molecules.

6. The method of any one of claims 1-5, wherein (i) the oligonucleotide molecule attached to the first of the two different binding molecules is the same as or different from the oligonucleotide molecule attached to the second of the two different binding molecules; and/or (ii) the further oligonucleotide molecules are identical or comprise at least two different oligonucleotides.

7. The method of any one of claims 1-6, wherein amplification is a rolling circle amplification.

8. The method of any one of claims 1-7, wherein the cell is fixed before or after the step of attaching the cell from the suspension of cells to the hydrophilic solid support.

9. The method of claim 7, wherein fixation is performed:
i. in ethanol, preferably 70%(v/v) ethanol;
ii. for between 1 h to 24 hrs, between 2 hrs to 12 hrs, or between 3 hrs to 6 hrs, preferably at least 2 hrs; and/or
iii. at a temperature of between -80 °C to 30 °C, between -40 °C to 25 °C, or between -20 °C to 25 °C.

10. A method for detection of nucleic acid end(s) in a circulating tumour cell, wherein the method comprises the steps of:
a) attaching a circulating tumour cell from a suspension of circulating tumour cells to a hydrophilic solid support under conditions such that the suspension of circulating tumour cells does not dry out;
b) adding nucleic acid binding molecules to the cell attached to the solid support under conditions such that the nucleic acid binding molecules bind to the nucleic acid end(s) in the cell;
c) adding two different binding molecules that bind to the nucleic acid binding molecules bound to the nucleic acid end(s), wherein each of the two different binding molecules is attached to an oligonucleotide molecule;
d) adding further oligonucleotide molecules which hybridise with the oligonucleotide molecules attached to the two different binding molecules to form a circular amplification template;
e) performing amplification to produce an amplification product; and
f) detecting the amplification product to detect the nucleic acid end(s) in the cell.

11. A method for detection of nucleic acid end(s) in a cell, wherein the method comprises the steps of:
a) attaching a cell from a suspension of cells to a hydrophilic solid support under conditions such that the suspension of cells does not dry out;
b) removing the suspension of cells to remove unattached cells;
c) fixing the cell attached to the solid support;
d) adding nucleic acid binding molecules to the cell attached to the solid support under conditions such that the nucleic acid binding molecules bind to the nucleic acid end(s) in the cell;
e) adding two different binding molecules that bind to the nucleic acid binding molecules bound to the nucleic acid end(s), wherein each of the two different binding molecules is attached to an oligonucleotide molecule;
f) adding further oligonucleotide molecules which hybridise with the oligonucleotide molecules attached to the two different binding molecules to form a circular amplification template;
g) performing amplification to produce an amplification product; and
h) detecting the amplification product to detect the nucleic acid end(s) in the cell.

12. A method for detection of nucleic acid end(s) in a cell, wherein the method comprises the steps of:
a) attaching a cell from a suspension of cells to a hydrophilic solid support under conditions such that the suspension of cells does not dry out;
b) removing the suspension of cells to remove unattached cells;
c) fixing the cell attached to the solid support;
d) storing the cell at a temperature of between -30 °C to -15°C for at least 4 hrs;
e) adding nucleic acid binding molecules to the cell attached to the solid support under conditions such that the nucleic acid binding molecules bind to the nucleic acid end(s) in the cell;
f) adding two different binding molecules that bind to the nucleic acid binding molecules bound to the nucleic acid end(s), wherein each of the two different binding molecules is attached to an oligonucleotide molecule;
g) adding further oligonucleotide molecules which hybridise with the oligonucleotide molecules attached to the two different binding molecules to form a circular amplification template;
h) performing amplification to produce an amplification product; and
i) detecting the amplification product to detect the nucleic acid end(s) in the cell.

13. A kit for detection of nucleic acid end(s) in a cell, the kit comprising:
a) a hydrophilic solid support;
b) nucleic acid binding molecules;
c) two different binding molecules that bind to the nucleic acid binding molecules;
d) further oligonucleotide molecules; and
e) reagents for performing amplification.

14. A method for assessing effectiveness of a therapeutic agent, the method comprising the steps:
a) performing the method of any one of claims 1-12 on a suspension of circulating tumour cells obtained from a subject before the administration of the therapeutic agent,
b) performing the method of any one of claims 1-12 on a suspension of circulating tumour cells obtained from the subject after the administration of the therapeutic agent,
c) comparing the amount of the nucleic acid end(s) detected in steps a) and b), wherein the therapeutic agent is effective if the amount of the nucleic acid end(s) is greater in step b) than step a),
wherein steps a) and b) can be performed in any order.

15. A method for assessing nucleic acid damage caused by an agent, the method comprising the steps:
a) performing the method of any one of claims 1-9 or claims 11 or 12 on a suspension of cells obtained from a subject before administration of, or exposure to, an agent,
b) performing the method of any one of claims 1-9 or claims 11 or 12 on a suspension of cells obtained from the subject after the administration of, or exposure to, the agent,
c) comparing the amount of the nucleic acid end(s) detected in steps a) and b), wherein the agent causes nucleic acid damage if the amount of the nucleic acid end(s) is greater in step b) than step a),
wherein steps a) and b) can be performed in any order.
